# EUROPÄISCHE PATENTSCHRIFT

(11) **EP 1 934 345 B1**
(45) Veröffentlichungstag und Bekanntmachung des Hinweises auf die Patenterteilung: **21.03.2012**
(21) Anmeldenummer: 06805937.7
(22) Anmeldetag: 28.09.2006
(51) Int. Cl.: C12N 15/09, C12N 15/00

(54) **MODIFIKATIONEN VON RNA, DIE ZU EINER ERHÖHTEN TRANSKRIPTSTABILITÄT UND TRANSLATIONSEFFIZIENZ FÜHREN**
MODIFICATION OF RNA, PRODUCING AN INCREASED TRANSCRIPT STABILITY AND TRANSLATION EFFICIENCY
MODIFICATIONS D'ARN, QUI PERMETTENT UNE STABILITE DE TRANSCRIPTION ET UNE EFFICACITE DE TRANSLATION AMELIOREES

(30) Priorität: 28.09.2005 DE 102005046490
(43) Veröffentlichungstag der Anmeldung: 25.06.2008
(60) Teilanmeldung: 10014762.8
(73) Patentinhaber: BioNTech AG, 55131 Mainz (DE)
(72) Erfinder: SAHIN, Ugur, 55116 Mainz (DE); HOLTKAMP, Silke, 45470 Mühlheim (DE); TÜRECI, Özlem, 55116 Mainz (DE); KREITER, Sebastian, 55131 Mainz (DE)
(74) Vertreter: Schnappauf, Georg
(86) Internationale Anmeldenummer: PCT/EP2006/009448
(87) Internationale Veröffentlichungsnummer: WO 2007/036366

(56) Entgegenhaltungen:
- TANGUAY R L ET AL: "Translational efficiency is regulated by the length of the 3' untranslated region" MOLECULAR AND CELLULAR BIOLOGY, AMERICAN SOCIETY FOR MICROBIOLOGY, WASHINGTON, US, Bd. 16, Nr. 1, 1996, Seiten 146-156, XP002370890 ISSN: 0270-7306
- GUHANIYOGI J ET AL: "Regulation of mRNA stability in mammalian cells" GENE, ELSEVIER, AMSTERDAM, NL, Bd. 265, Nr. 1-2, 7. März 2001 (2001-03-07), Seiten 11-23, XP004230718 ISSN: 0378-1119
- GALLIE D R: "A tale of two termini: - A functional interaction between the termini of an mRNA is a prerequisite for efficient translation initiation" GENE, ELSEVIER, AMSTERDAM, NL, Bd. 216, Nr. 1, August 1998 (1998-08), Seiten 1-11, XP004149275 ISSN: 0378-1119
- LI X ET AL: "GENERATION OF DESTABILIZED GREEN FLUORESCENT PROTEIN AS A TRANSCRIPTION REPORTER" JOURNAL OF BIOLOGICAL CHEMISTRY, AMERICAN SOCIETY OF BIOLOCHEMICAL BIOLOGISTS, BIRMINGHAM,, US, Bd. 273, Nr. 52, 25. Dezember 1998 (1998-12-25), Seiten 34970-34975, XP000984126 ISSN: 0021-9258
- YU JIA ET AL: "Structural and functional analysis of an mRNP complex that mediates the high stability of human beta-globin mRNA" MOLECULAR AND CELLULAR BIOLOGY, Bd. 21, Nr. 17, September 2001 (2001-09), Seiten 5879-5888, XP002418544 ISSN: 0270-7306
- PESOLE G ET AL: "Structural and functional features of eukaryotic mRNA untranslated regions" GENE: AN INTERNATIONAL JOURNAL ON GENES AND GENOMES, ELSEVIER, AMSTERDAM, NL, Bd. 276, Nr. 1-2, 3. Oktober 2001 (2001-10-03), Seiten 73-81, XP004308679 ISSN: 0378-1119
- HOLTKAMP SILKE ET AL: "Modification of antigen-encoding RNA increases stability, translational efficacy, and T-cell stimulatory capacity of dendritic cells." BLOOD 15 DEC 2006, Bd. 108, Nr. 13, 15. Dezember 2006 (2006-12-15), Seiten 4009-4017, XP002418545 ISSN: 0006-4971
- BOCZKOWSKID ET AL: "INDUCTION OF TUMOR IMMUNITY AND CYTOTOXIC T LYMPHOCYTE RESPONSES USING DENDRITIC CELLS TRANSFECTED WITH MESSENGER RNA AMPLIFIED FROM TUMOR CELLS" CANCER RESEARCH, AMERICAN ASSOCIATION FOR CANCER RESEARCH, BALTIMORE, MD, US, Bd. 60, 15. Februar 2000 (2000-02-15), Seiten 1028-1034, XP001048606 ISSN: 0008-5472
- STRONG T V ET AL: "INCORPORATION OF BETA-GLOBIN UNTRANSLATED REGIONS INTO A SINDBIS VIRUS VECTOR FOR AUGMENTATION OF HETEROLOGOUS MRNA EXPRESSION" GENE THERAPY, MACMILLAN PRESS LTD., BASINGSTOKE, GB, Bd. 4, Nr. 6, Juni 1997 (1997-06), Seiten 624-627, XP009012148 ISSN: 0969-7128
- PREISS T ET AL: "Dual function of the messenger RNA cap structure in poly(A)-tail-promoted translation in yeast" NATURE, NATURE PUBLISHING GROUP, LONDON, GB, Bd. 392, Nr. 6675, 2. April 1998 (1998-04-02), Seiten 516-520, XP002240279 ISSN: 0028-0836
- CARRALOT J-P ET AL: "Polarization of immunity induced by direct injection of naked sequence-stabilized mRNA vaccines" CMLS CELLULAR AND MOLECULAR LIFE SCIENCES, BIRKHAUSER VERLAG, HEIDELBERG, DE, Bd. 61, Nr. 18, September 2004 (2004-09), Seiten 2418-2424, XP002355208 ISSN: 1420-682X
- CONRY R M ET AL: "CHARACTERIZATION OF A MESSENGER RNA POLYNUCLEOTIDE VACCINE VECTOR" CANCER RESEARCH, AMERICAN ASSOCIATION FOR CANCER RESEARCH, BALTIMORE, MD, US, Bd. 55, Nr. 7, 1. April 1995 (1995-04-01), Seiten 1397-1400, XP000576177 ISSN: 0008-5472
- TEUFEL R ET AL: "Human peripheral blood mononuclear cells transfected with messenger RNA stimulate antigen-specific cytotoxic T-lymphocytes in vitro" CMLS CELLULAR AND MOLECULAR LIFE SCIENCES, BIRKHAUSER VERLAG, HEIDELBERG, DE, Bd. 62, Nr. 15, August 2005 (2005-08), Seiten 1755-1762, XP002392461 ISSN: 1420-682X
- MIGNONE F ET AL: "Untranslated regions of mRNAs" GENOME BIOLOGY 2002 UNITED KINGDOM, Bd. 3, Nr. 3, 2002, Seiten 0004.1-0004.10, XP002428984 ISSN: 1465-6906

## Beschreibung

Konventionelle Impfstoffe, einschließlich der attenuierten oder inaktivierten Pathogene, sind in vielen Bereichen wirksam, vermitteln aber dennoch gegen einige infektiöse Pathogene und Tumore keine wirksam schützende Immunität. Es sind Impfstoffe erforderlich, die wirksam, vielseitig, schnell und günstig in der Herstellung und einfach zu lagern sind.
Nachdem gezeigt werden konnte, dass die direkte intramuskuläre Injektion von Plasmid-DNA zu langanhaltender Expression der kodierten Gene auf der Zelloberfläche führt (Wolff et al., 1990), wurden auf DNA basierte Impfstoffe als neue, vielversprechende Immunisierungsstrategie angesehen. Dies war ein bedeutender Ansporn, um auf Nukleinsäuren basierte Impfstoffe zu entwickeln. Zunächst wurden auf DNA basierte Impfstoffe gegen infektiöse Pathogene erprobt (Cox et al., 1993; Davis et al., 1993; Ulmer et al., 1993; Wang et al., 1993), bald aber auch in der Gentherapie gegen Tumore weiter erforscht, um eine spezifische Antitumor-Immunität zu induzieren (Conry et al., 1994; Conry et al., 1995a; Spooner et al., 1995; Wang et al., 1995). Diese Strategie der Tumorimmunisierung hat eine Reihe von entscheidenden Vorteilen. Auf Nukleinsäure basierte Impfstoffe sind einfach in der Herstellung und relativ kostengünstig. Sie können außerdem aus einer geringen Zellzahl amplifiziert werden.
DNA ist stabiler als RNA, birgt aber einige potentielle Sicherheitsrisiken wie die Induktion von anti-DNA-Antikörpern (Gilkeson et al., 1995) und die Integration des Transgens ins Wirtsgenom. Dies kann zur Inaktivierung zellulärer Gene, einer unkontrollierbaren Langzeit-Expression des Transgens oder Onkogenese führen und ist daher für tumorassoziierte Antigene mit onkogenem Potential wie z.B. erb-B2 (Bargmann et al., 1986) und p53 (Greenblatt et al., 1994) im Allgemeinen nicht anwendbar. Um diese potentiellen Gefahren zu umgehen, bietet der Gebrauch von RNA eine attraktive Alternative.
Zu den Vorteilen der Verwendung von RNA als einer Art reversiblen Gentherapie zählt die vorübergehende Expression und der nicht transformierende Charakter. Die RNA muss nicht in den Kern gelangen, um transgen exprimiert zu werden und kann außerdem nicht ins Wirtsgenom integrieren, wodurch das Risiko einer Onkogenese eliminiert ist. Wie mit DNA (Condon et al., 1996; Tang et al., 1992) können auch durch die Injektion von RNA sowohl die zelluläre als auch die humorale Immunantwort in vivo induziert werden (Hoerr et al., 2000; Ying et al., 1999).

Für die Immuntherapie mit in vitro transkribierter RNA (IVT-RNA) werden zwei unterschiedliche Strategien verfolgt, die beide in verschiedenen Tiermodellen erfolgreich getestet werden konnten. Entweder wird die RNA über unterschiedliche Immunisierungsrouten direkt injiziert (Hoerr et al., 2000) oder Dendritische Zellen (DCs) werden durch Lipofektion oder Elektroporation mit in vitro transkribierter RNA transfiziert und dann appliziert (Heiser et al., 2000). In kürzlich erschienenen Studien konnte gezeigt werden, dass die Immunisierung mit RNA-transfizierten DCs antigenspezifische cytotoxische T-Lymphozyten (CTL) in vitro und in vivo induziert (Su et al., 2003; Heiser et al., 2002). Für die optimale Induktion der T-Zell-vermittelten Immunantworten ist unter anderem die Dosis, d.h. die Dichte der Antigen-Präsentation auf den DCs von zentraler Bedeutung.
Mit dem Ziel, eine verlängerte Expression von transferierter IVT-RNA und damit eine erhöhte Antigenpräsentation auf DCs zu erreichen, wurde versucht, IVT-RNA durch verschiedene Modifikationen zu stabilisieren. Eine Grundvoraussetzung für die Translation ist das Vorhandensein einer 3'-Poly(A)-Sequenz, wobei die Translationseffizienz mit der Poly(A)-Länge korreliert (Preiss and Hentze, 1998). Durch das 5'-Cap und die 3'-Poly(A)-Sequenz wird die Translation in vivo synergistisch aktiviert (Gallie, 1991). Nicht-translatierte Regionen (UTR) von Globin-Genen sind weitere bekannte Elemente, die zur Stabilisierung von RNA und zu einer Erhöhung der Translationseffizienz beitragen können (Malone et al., 1989).
Aus der Literatur sind einige IVT-Vektoren bekannt, die standardisiert als Matrize für die in vitro-Transkription genutzt werden und gentechnisch so modifiziert worden sind, dass stabilisierte RNA-Transkripte resultieren. Aktuell in der Literatur beschriebene Protokolle (Conry et al., 1995b; Teufel et al., 2005; Strong et al., 1997; Carralot et al., 2004; Boczkowski et al., 2000) basieren auf einem Plasmid-Vektor mit folgender Struktur: ein 5'-gelegener RNA-Polymerase-Promoter, der die Transkription von RNA ermöglicht, gefolgt vom einem interessierenden Gen ("gene of interest"), das entweder 3' und/oder 5' von nicht-translatierten Regionen (UTR) flankiert wird und 3' eine Polyadenylkassette mit 50-70 A-Nukleotiden. Vor der in vitro Transkription wird das zirkuläre Plasmid durch Restriktionsenzyme vom Typ II (Erkennungssequenz entspricht der Schnittstelle) hinter der Polyadenylkassette linearisiert. Die Polyadenylkassette entspricht damit der späteren Poly(A)-Sequenz im Transkript. Als Folge dieses Vorgehens bleiben einige Nukleotide als Teil der Enzymschnittstelle nach der Linearisierung zurück und verlängern bzw. verdecken die Poly(A)-Sequenz am 3' Ende. Unklar ist, ob dieser unphysiologische Überhang einen Einfluss auf die Menge des Proteins hat, welches intrazellulär von einem solchen Konstrukt gebildet wird.

Somit scheint RNA besonders gut für klinische Anwendungen geeignet zu sein. Der Gebrauch von RNA in der Gentherapie wird aber vor allem durch die kurze Halbwertszeit von RNA insbesondere im Zytoplasma stark eingeschränkt, was eine geringe Proteinexpression zur Folge hat.

Es war die Aufgabe der vorliegenden Erfindung, RNA mit erhöhter Stabilität und Translationseffizienz und Mittel für eine Gewinnung derartiger RNA bereitzustellen. Durch Verwendung dieser RNA bei gentherapeutischen Ansätzen sollen gesteigerte Expressionsraten erzielbar sein.

Diese Aufgabe wird erfindungsgemäß durch den Gegenstand der Patentansprüche gelöst

Die vorliegende Erfindung betrifft eine Stabilisierung von RNA, insbesondere mRNA, und eine Steigerung der Translation von mRNA. Insbesondere betrifft die vorliegende Erfindung drei Modifikationen von RNA, insbesondere in vitro transkribierter RNA, die zu einer erhöhten Transkriptstabilität und Translationseffizienz führen.

Erfindungsgemäß wurde festgestellt, dass RNA mit einer offen endenden Poly(A)-Sequenz effizienter translatiert wird als RNA mit einer verdeckt endenden Poly(A)-Sequenz. Ferner wurde festgestellt, das eine lange Poly(A)-Sequenz, insbesondere von etwa 120 bp, zu einer optimalen Transkriptstabilität und Translationseffizienz von RNA führt. Auch konnte erfindungsgemäß gezeigt werden, dass eine doppelte 3'-nicht translatierte Region (UTR), insbesondere des humanen Beta-Globin-Gens, in einem RNA-Molekül zu einer Verbesserung der Translationseffizienz führt, die über den Summationseffekt, der mit zwei einzelnen UTR zu erwarten ist, deutlich hinaus geht. Erfindungsgemäß stellte sich heraus, dass eine Kombination der vorstehend beschriebenen Modifikationen einen synergistischen Einfluss auf die Stabilisierung der RNA und die Erhöhung der Translation hat.

Unter Verwendung von quantitativer RT-PCR und eGFP-Varianten, um Transkriptmengen und Proteinausbeute zu messen, konnte erfindungsgemäß gezeigt werden, dass die erfindungsgemäßen RNA-Modifikationen unabhängig die RNA-Stabilität und Translationseffizienz bei Transfektion von dendritischen Zellen (DC) verstärken. Somit konnte die Dichte von Antigen-spezifisches Peptid/MHC-Komplexen auf den transfizierten Zellen, sowie ihre Fähigkeit, Antigen-spezifische CD4⁺-, sowie CD8⁺-T-Zellen zu stimulieren und expandieren, erhöht werden. Die Erfindung betrifft daher eine Strategie zur Optimierung von RNA-transfizierten DC-Vakzinen durch Verwendung von RNA, die durch die erfindungsgemäß beschriebenen RNA-Modifikationen modifiziert wurde.

Vorzugsweise wird erfindungsgemäß eine Modifizierung und dadurch Stabilisierung und/oder Steigerung der Translationseffizienz von RNA dadurch erreicht, dass Expressionsvektoren gentechnisch modifiziert werden, die vorzugsweise als Matrize für die in vitro-Transkription von RNA dienen.

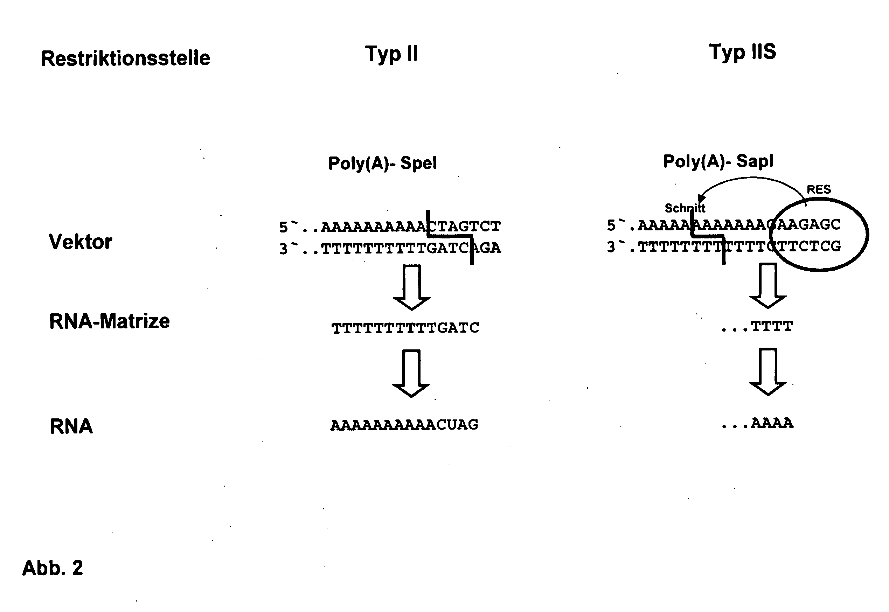

Solche Vektoren sollen insbesondere die Transkription von RNA mit einer Poly(A)-Sequenz erlauben, wobei die Poly(A)-Sequenz vorzugsweise in der RNA ein offenes Ende aufweist, d.h. keine von A-Nukleotiden verschiedene Nukleotide die Poly(A)-Sequenz an ihrem 3'-Ende flankieren. Eine offen endende Poly(A)-Sequenz in der RNA kann dadurch erreicht werden, dass in einen Expressionsvektor, welcher die Transkription von RNA unter der Kontrolle eines 5' gelegenen RNA-Polymerase-Promotors erlaubt und eine Polyadenylkassette (Poly(A)-Sequenz) enthält, eine Restriktionsschnittstelle vom Typ IIs eingebracht wird, wobei die Erkennungssequenz 3' von der Poly(A)-Sequenz liegt, während die Schnittstelle stromaufwärts und damit innerhalb der Poly(A)-Sequenz liegt. Durch Restriktionsspaltung an der Restriktionsschnittstelle vom Typ IIS wird bei einem Plasmid eine Linearisierung des Plasmids innerhalb der Poly(A)-Sequenz möglich (Abb. 2). Das linearisierte Plasmid kann dann als Matrize für eine in vitro-Transkription verwendet werden, wobei das resultierende Transkript mit einer unverdeckten Poly(A)-Sequenz endet.

Weiterhin kann eine Modifizierung und dadurch Stabilisierung und/oder Steigerung der Translationseffizienz von RNA dadurch erreicht werden, dass Expressionsvektoren gentechnisch derart modifiziert werden, dass sie die Transkription von RNA mit zwei oder mehreren 3'-nicht translatierten Regionen an ihrem 3'-Ende und vorzugsweise zwischen der für ein Peptid oder Protein kodierenden Sequenz (offenes Leseraster) und der Poly(A)-Sequenz erlauben.

In einem Aspekt betrifft die Erfindung ein Nukleinsäuremolekül, umfassend in 5'→3'-richtung der Transkription: (a) einen Promotor, (b) eine transkribierbare Nukleinsäuresequenz oder eine Nukleinsäuresequenz für ein Einbringen einer transkribierbaren Nukleinsäuresequenz, (c-1) eine erste Nukleinsäuresequenz, (c-2) eine zweite Nukleinsäuresequenz, und gegebenenfalls (c-3) mindestens eine weitere Nukleinsäuresequenz,
wobei die Nukleinsäuresequenzen (c-1), (c-2) und gegebenenfalls (c-3) ausgewählt sind aus der Gruppe bestehend aus:
(I) einer Nukleinsäuresequenz, die der 3'-nicht-translatierten Region eines Globin-Gens entspricht, und
(II) einer Nukleinsäuresequenz, die zu der Nukleinsäuresequenz unter (I) mindestens 90% Identität aufweist,
wobei die Nukleinsäuresequenzen (c-1), (c-2) und gegebenenfalls (c-3) unabhängig voneinander von einem Gen abgeleitet sind, ausgewählt aus der Gruppe, bestehend aus alpha2-Globin-Gen, alpha1-Globin-Gen, und beta-Globin-Gen, und
wobei die Nukleinsäuresequenzen (b), (c-1), (c-2) und gegebenenfalls (c-3) unter Kontrolle des Promotors (a) in ein gemeinsames Transkript transkribierbar sind und in dem gemeinsamen Transkript die von den Nukleinsäuresequenzen (c-1), (c-2) und gegebenenfalls (c-3) transkribierten Nukleinsäuresequenzen wirksam sind, die Translationseffizienz und/oder die Stabilität der von der transkribierbaren Nukleinsäuresequenz (b) transkribierten Nukleinsäuresequenz zu erhöhen.

Die Nukleinsäuresequenzen (c-1), (c-2) und gegebenenfalls (c-3) können gleich oder verschieden sein.

In einer Ausführungsform umfasst das Nukleinsäuremolekül ferner (d) eine Nukleinsäuresequenz, die bei einer Transkription unter Kontrolle des Promotors (a) für eine Nukleotidsequenz von mindestens 20 aufeinander folgenden A-Nukleotiden in dem Transkript kodiert.

Die Nukleinsäuresequenzen (b), (c-1), (c-2), gegebenenfalls (c-3), und (d) sind unter Kontrolle des Promotors (a) vorzugsweise in ein gemeinsames Transkript transkribierbar und in dem gemeinsamen Transkript sind die von den Nukleinsäuresequenzen (c-1) und/oder (c-2) und/oder gegebenfalls (c-3) und/oder (d) transkribierten Nukleinsäuresequenzen vorzugsweise wirksam, die Translationseffizienz und/oder die Stabilität der von der transkribierbaren Nukleinsäuresequenz (b) transkribierten Nukleinsäuresequenz zu erhöhen.

In bestimmten Ausführungsformen kodiert die Nukleinsäuresequenz (d) bei einer Transkription unter Kontrolle des Promotors (a) für eine Nukleotidsequenz von mindestens 40, vorzugsweise mindestens 80, vorzugsweise mindestens 100 und insbesondere etwa 120 aufeinander folgenden A-Nukleotiden in dem Transkript. Vorzugsweise kodiert die Nukleinsäuresequenz (d) bei einer Transkription unter Kontrolle des Promotors (a) für eine Nukleotidsequenz von bis zu 500, vorzugsweise bis zu 400, vorzugsweise bis zu 300, vorzugsweise bis zu 200 und insbesondere bis zu 150 aufeinander folgenden A-Nukleotiden in dem Transkript.

In einer Ausführungsform ist das Nukleinsäuremolekül dadurch gekennzeichnet, dass es innerhalb der Nukleinsäuresequenz (d) vorzugsweise enzymatisch oder anderweitig biochemisch derart spaltbar ist, dass durch die Spaltung ein Nukleinsäuremolekül entsteht, das in 5'→3'-Richtung der Transkription den Promotor (a), die Nukleinsäuresequenz (b), die Nukleinsäuresequenzen (c-1), (c-2) und gegegenenfalls (c-3), und zumindest einen Teil der Nukleinsäuresequenz (d) umfasst, wobei der zumindest eine Teil der Nukleinsäuresequenz (d) bei einer Transkription unter Kontrolle des Promotors (a) für eine Nukleotidsequenz von mindestens 20 aufeinander folgenden A-Nukleotiden in dem Transkript kodiert und das Transkript als 3'-terminales Nukleotid ein A-Nukleotid aus der Nukleotidsequenz von mindestens 20 aufeinander folgenden A-Nukleotiden aufweist.

Vorzugsweise weist das Nukleinsäuremolekül nach der Spaltung am Ende des Stranges, der als Matrize für die Nukleotidsequenz von mindestens 20 aufeinander folgenden A-Nukleotiden dient, ein T-Nukleotid auf, das Teil der Nukleotidsequenz ist, die als Matrize für die Nukleotidsequenz von mindestens 20 aufeinander folgenden A-Nukleotiden in dem Transkript dient.

In bestimmten Ausführungsformen kodiert der zumindest Teil der Nukleinsäuresequenz (d) bei einer Transkription unter Kontrolle des Promotors (a) für eine Nukleotidsequenz von mindestens 40, vorzugsweise mindestens 80, vorzugsweise mindestens 100 und insbesondere etwa 120 aufeinander folgenden A-Nukleotiden in dem Transkript. Vorzugsweise kodiert die Nukleinsäuresequenz (d) bei einer Transkription unter Kontrolle des Promotors (a) für eine Nukleotidsequenz von bis zu 500, vorzugsweise bis zu 400, vorzugsweise bis zu 300, vorzugsweise bis zu 200 und insbesondere bis zu 150 aufeinander folgenden A-Nukleotiden in dem Transkript.

Das erfindungsgemäße Nukleinsäuremolekül liegt vor der Spaltung vorzugsweise als kreisförmig geschlossenes Molekül und nach der Spaltung als lineares Molekül vor.

Vorzugsweise erfolgt die Spaltung mit Hilfe einer Restriktionsschnittstelle, wobei die Restriktionsschnittstelle vorzugsweise eine Restriktionsschnittstelle für eine Restriktions-Endonuklease des Typs IIS ist.

Die Erkennungssequenz für die Restriktions-Endonuklease des Typs IIS liegt in einer Ausführungsform stromabwärts und 5-26 Basenpaare, vorzugsweise 24-26 Basenpaare entfernt vom 3'-Ende der Nukleinsäuresequenz (d).

In einer bevorzugten Ausführungsform ist das beta-Globin-Gen humanes beta-Globin-Gen.

In einer bevorzugten Ausführungsform entspruchen die Nukleinsäuresequenzen (c-1), (c-2) und gegebenenfalls (c-3) unabhängig voneinander der Nukleinsäuresequenz gemäß SEQ ID NO: 1 des Sequenzprotokolls oder einer davon abgeleiteten Nukleinsäuresequenz.

In einem erfindungsgemäßen Nukleinsäuremolekül umfasst die transkribierbare Nukleinsäuresequenz vorzugsweise eine für ein Peptid oder Protein kodierende Nukleinsäuresequenz und die Nukleinsäuresequenz für ein Einbringen einer transkribierbaren Nukleinsäuresequenz ist vorzugsweise eine multiple Klonierungsstelle.

Ferner kann ein erfindungsgemäßes Nukleinsäuremolekül ein oder mehrere Mitglieder umfassen, ausgewählt aus der Gruppe bestehend aus: (i) einem Reportergen; (ii) einem selektierbaren Marker; und (iii) einem Replikationsursprung.

Ein erfindungsgemäßes Nukleinsäuremolekül liegt in einer Ausführungsform in einer kreisförmig geschlossenen Konformation vor und ist vorzugsweise insbesondere nach einer Linerarisierung für die in vitro-Transkription von RNA, insbesondere mRNA, geeignet.

In weiteren Aspekten betrifft die Erfindung ein Nukleinsäuremolekül, das durch Linearisierung eines vorstehend beschriebenen Nukleinsäuremoleküls, vorzugsweise durch Spaltung innerhalb der Nukleinsäuresequenz, die für eine Nukleotidsequenz von mindestens 20 aufeinander folgenden A-Nukleotiden kodiert, erhältlich ist, und RNA, die durch Transkription, vorzugsweise in vitro-Transkription, mit vorstehend beschriebenen Nukleinsäuremolekülen unter Kontrolle des Promotors (a) erhältlich ist, wobei in der RNA Transkripte der Nukleinsäuresequenzen (b), (c-1), (c-2) und gegebenenfalls (c-3) in einem gemeinsamen Transkript vorliegen.

In einem weiteren Aspekt betrifft die Erfindung ein Verfahren zur in vitro-Transkription eines ausgewählten RNA-Moleküls, um dessen Stabilität und/oder Translationseffizienz zu erhöhen, umfassend: (i) Koppeln einer ersten Nukleinsäuresequenz (b-1) an dem 3'-Ende einer Nukleinsäuresequenz (a), die in das RNA-Molekül transkribierbar ist, (ii) Koppeln einer zweiten Nukleinsäuresequenz (b-2) an dem 3'-Ende der ersten Nukleinsäuresequenz (b-1), und gegebenenfalls (iii) Koppeln mindestens einer weiteren Nukleinsäuresequenz (b-3) an dem 3'-Ende der zweiten Nukleinsäuresequenz (b-2),
wobei die Nukleinsäuresequenzen (b-1), (b-2) und gegebenenfalls (b-3) ausgewählt sind aus der Gruppe bestehend aus:
(I) einer Nukleinsäuresequenz, die der 3'-nicht-translatierten Region eines Globin-Gens entspricht und
(II) einer Nukleinsäuresequenz, die zu der Nukleinsäuresequenz unter (I) mindestens 90% Identität aufweist,
wobei die Nukleinsäuresequenzen (b-1), (b-2) und gegebenenfalls (b-3) unabhängig voneinander von einem Gen abgeleitet sind, ausgewählt aus der Gruppe, bestehend aus alpha2-Globin-Gen, alpha1-Globin-Gen, und beta-Globin-Gen, und
(iv) in vitro-Transkription der erhaltenen Nukleinsäure,
wobei die Nukleinsäuresequenzen (a), (b-1), (b-2) und gegebenenfalls (b-3) in ein gemeinsames Transkript transkribierbar sind und in dem gemeinsamen Transkript die von den Nukleinsäuresequenzen (b-1), (b-2) und gegebenenfalls (b-3) transkribierten Nukleinsäuresequenzen wirksam sind, die Translationseffizienz und/oder die Stabilität der von der transkribierbaren Nukleinsäuresequenz (a) transkribierten Nukleinsäuresequenz zu erhöhen.

In einem weiteren Aspekt betrifft die Erfindung ein Verfahren zur Translation eines ausgewählten mRNA-Moleküls, um dessen Expression zu erhöhen, umfassend: (i) Koppeln einer ersten Nukleinsäuresequenz (b-1) an dem 3'-Ende einer Nukleinsäuresequenz (a), die in das mRNA-Molekül transkribierbar ist, (ii) Koppeln einer zweiten Nukleinsäuresequenz (b-2) an dem 3'-Ende der ersten Nukleinsäuresequenz (b-1), und gegebenenfalls (iii) Koppeln mindestens einer weiteren Nukleinsäuresequenz (b-3) an dem 3'-Ende der zweiten Nukleinsäuresequenz (b-2),
wobei die Nukleinsäuresequenzen (b-1), (b-2) und gegebenenfalls (b-3) ausgewählt sind aus der Gruppe bestehend aus:
(I) einer Nukleinsäuresequenz, die der 3'-nicht-translatierten Region eines Globin-Gens entspricht, oder
(II) einer Nukleinsäuresequenz, die zu der Nukleinsäuresequenz unter (I) mindestens 90% Identität aufweist,
wobei die Nukleinsäuresequenzen (b-1), (b-2) und gegebenenfalls (b-3) unabhängig voneinander von einem Gen abgeleitet sind, ausgewählt aus der Gruppe, bestehend aus alpha2-Globin-Gen, alpha1-Globin-Gen, und beta-Globin-Gen, und
(iv) Translatieren der mRNA, die durch Transkription der erhaltenen Nukleinsäure erhältlich ist,
wobei die Nukleinsäuresequenzen (a), (b-1), (b-2) und gegebenenfalls (b-3) in ein gemeinsames Transkript transkribierbar sind und in dem gemeinsamen Transkript die von den Nukleinsäuresequenzen (b-1), (b-2) und gegebenenfalls (b-3) transkribierten Nukleinsäuresequenzen wirksam sind, die Translationseffizienz und/oder die Stabilität der von der transkribierbaren Nukleinsäuresequenz (a) transkribierten Nukleinsäuresequenz zu erhöhen. Vorzugsweise erfolgt die Transkription in vitro.

Erfindungsgemäß betrifft der Begriff "Koppeln einer Nukleinsäuresequenz an dem 3'-Ende einer Nukleinsäuresequenz" eine kovalente Verbindung der zwei Nukleinsäuresequenzen, so dass die erste Nukleinsäuresequenz stromabwärts zu der zweiten Nukleinsäuresequenz liegt und von dieser durch zusätzliche Nukleinsäuresequenzen getrennt sein kann.

In einer weiteren Ausführungsform umfassen die erfindungsgemäßen Verfahren ferner ein Koppeln einer Nukleinsäuresequenz (c), die bei einer Transkription für eine Nukleotidsequenz von mindestens 20 aufeinander folgenden A-Nukleotiden kodiert, an dem 3'-Ende der Nukleinsäuresequenz (b-2) oder gegebenenfalls der Nukleinsäursequenz (b-3).

Die Nukleinsäuresequenzen (a), (b-1), (b-2) und gegebenenfalls (b-3), und (c) sind vorzugsweise in ein gemeinsames Transkript transkribierbar und in dem gemeinsamen Transkript sind die von den Nukleinsäuresequenzen (b-1) und/oder (b-2) und/oder gegebenenfalls (b-3) und/oder (c) transkribierten Nukleinsäuresequenzen vorzugsweise wirksam, die Translationseffizienz und/oder die Stabilität der von der Nukleinsäuresequenz (a) transkribierten Nukleinsäuresequenz zu erhöhen.

In bestimmten Ausführungsformen kodiert die Nukleinsäuresequenz (c) bei einer Transkription für eine Nukleotidsequenz von mindestens 40, vorzugsweise mindestens 80, vorzugsweise mindestens 100 und insbesondere etwa 120 aufeinander folgenden A-Nukleotiden in dem Transkript. Vorzugsweise kodiert die Nukleinsäuresequenz (c) bei einer Transkription für eine Nukleotidsequenz von bis zu 500, vorzugsweise bis zu 400, vorzugsweise bis zu 300, vorzugsweise bis zu 200 und insbesondere bis zu 150 aufeinander folgenden A-Nukleotiden in dem Transkript.

In bestimmten Ausführungsformen umfassen die erfindungsgemäßen Verfahren ferner vor einer Transkription der erhaltenen Nukleinsäure eine Spaltung innerhalb der Nukleinsäuresequenz (c), so dass bei einer Transkription der so erhaltenen Nukleinsäure ein Transkripte erzeugt wird, das die von den Nukleinsäuresequenzen (a), (b-1), (b-2) und gegebenenfalls (b-3) transkribierten Nukleinsäuresequenzen und an seinem 3'-Ende eine Nukleotidsequenz von mindestens 20 aufeinander folgenden A-Nukleotiden aufweist, wobei das Transkript als 3'-terminals Nukleotid ein A-Nukleotid aus der Nukleotidsequenz von mindestens 20 aufeinander folgenden A-Nukleotiden aufweist.

In bestimmten Ausführungsformen weist das Transkript an seinem 3'-Ende eine Nukleotidsequenz von mindestens 40, vorzugsweise mindestens 80, vorzugsweise mindestens 100 und insbesondere etwa 120 aufeinander folgenden A-Nukleotiden auf. Vorzugsweise weist das Transkript an seinem 3'-Ende eine Nukleotidsequenz von bis zu 500, vorzugsweise bis zu 400, vorzugsweise bis zu 300, vorzugsweise bis zu 200 und insbesondere bis zu 150 aufeinander folgenden A-Nukleotiden auf.

In einer bevorzugten Ausführungsform ist das beta-Globin-Gen humanes beta-Globin-Gen.

In einer bevorzugten Ausführungform entsprechen die Nukleinsäuresequenzen (b-1), (b-2) und gegebenenfalls (b-3) unabhängig voneinander der Nukleinsäuresequenz gemäß SEQ ID NO: 1 des Sequenzprotokolls oder einer davon abgeleiteten Nukleinsäuresequenz.

Die Spaltung erfolgt in den erfindungsgemäßen Verfahren gemäß aller Aspekte vorzugsweise mit Hilfe einer Restriktionsschnittstelle, die vorzugsweise eine Restriktionsschnittstelle für eine Restriktions-Endonuklease des Typs IIS ist.

In einer Ausführungsform liegt die Erkennungssequenz für die Restriktions-Endonuklease des Typs IIS stromabwärts und 5-26 Basenpaare, vorzugsweise 24-26 Basenpaare, entfernt vom 3'-Ende der Nukleinsäuresequenz, die bei einer Transkription für eine Nukleotidsequenz von mindestens 20 aufeinander folgenden A-Nukleotiden kodiert.

Die Erfindung betrifft auch RNA, die durch die erfindungsgemäßen Verfahren zur in vitro-Transkription eines ausgewählten RNA-Moleküls erhältlich ist wobei in der RNA Transkripte der Nukleinsäuresequenzen (a), (b-1), (b-2) und gegebenenfalls (b-3) in einem gemeinsamen Transkript vorliegen, Vorzugsweise ist die RNA-Präparation, die durch die erfindungsgemäßen Verfahren zur in vitro-Transkription eines ausgewählten RNA-Moleküls von einem erfindungsgemäßen Nukleinsäuremolekül als Matrize erhältlich ist, bezüglich der Länge der Poly(A)-Sequenz der RNA homogen oder im wesentlichen homogen, d.h. in mehr als 90%, vorzugsweise mehr als 95%, vozugsweise mehr als 98% oder 99% der in der Präparation vorhandenen RNA-Moleküle unterscheidet sich die Länge der Poly(A)-Sequenz um nicht mehr als 10, vorzugsweise nicht mehr als 5, 4, 3, 2 oder 1 A-Nukleotide.

Die Erfindung kann beispielsweise zur Erhöhung der Expression rekombinanter Proteine bei einer zellulären Transkription und Expression genutzt werden. Insbesondere können bei einer Produktion von rekombinanten Proteinen die erfindungsgemäß beschriebenen Modifikationen sowie eine Kombination davon in Expressionsvektoren eingebracht werden und dazu genutzt werden, die Transkription von rekombinanten Nukleinsäuren und Expression von rekombinanten Proteinen in zellbasierten Systemen zu erhöhen. Hierzu zählt z.B. die Herstellung von rekombinanten Antikörpern, Hormonen, Zytokinen, Enzymen u.a. Dies erlaubt unter anderem die Senkung von Produktionskosten.

Ferner können die erfindungsgemäß beschriebenen Modifikationen sowie eine Kombination davon für gentherapeutische Anwendungen genutzt werden. Die Modifikationen können in Gentherapievektoren eingebracht werden und somit für eine Erhöhung der Expression eines Transgens genutzt werden. Hierfür können jegliche auf Nukleinsäuren (DNA/RNA) basierende Vektorsysteme (z.B. Plasmide, Adenoviren, Poxvirusvektoren, Influenzavirusvektoren, Alphavirusvektoren u.a.) verwendet werden. Die Transfektion von Zellen mit diesen Vektoren kann in vitro, z.B. in Lymphozyten oder Dendritische Zellen, oder auch in vivo durch direkte Applikation erfolgen.

Ferner kann durch die erfindungsgemäß beschriebenen Modifikationen sowie eine Kombination davon die Stabilität und/oder Expressionseffizienz von Ribonukleinsäuren und somit die Menge der von diesen Ribonukleinsäuren kodierten Peptide oder Proteine gesteigert werden. Kodierende Ribonukleinsäuren können beispielsweise zur transienten Expression von Genen eingesetzt werden, wobei mögliche Anwendungsgebiete auf RNA basierende Impfstoffe, die in vitro in Zellen transfiziert bzw. direkt in vivo appliziert werden, eine transiente Expression von funktionellen rekombinanten Proteinen in vitro, z.B. um Differenzierungsvergänge in Zellen zu initiieren oder Funktionen von Proteinen zu untersuchen, und eine transiente Expression von funktionellen rekombinanten Proteinen wie Erythropoietin, Hormone, Gerinnungshemmer etc., in vivo, insbesondere als Pharmazeutika, sind.

RNA, insbesondere in vitro transkribierte RNA, die durch die erfindungsgemäß beschriebenen Modifikationen modifiziert ist, kann insbesondere zur Transfektion von Antigen-präsentierenden Zellen und somit als Werkzeug für ein Zufuhr des zu präsentierenden Antigens und zur Beladung von Antigen-präsentierenden Zellen verwendet werden, wobei das zu präsentierende Antigen dem von der RNA exprimierten Peptid oder Protein entspricht oder davon insbesondere durch intrazelluläre Prozessierung wie Spaltung abgeleitet ist, d.h. das zu präsentierende Antigen ist beispielsweise eine Fragment des von der RNA exprimierten Peptids oder Proteins. Solche Antigen-präsentierenden Zellen können zur Stimulierung von T-Zellen, insbesondere CD4⁺- und/oder CD8⁺-T-Zellen, verwendet werden.

### Detaillierte Beschreibung der Erfindung

Erfindungsgemäß können Standardverfahren für eine Herstellung von rekombinanten Nukleinsäuren, Kultivierung von Zellen und Einbringen von Nukleinsäuren, insbesondere RNA, in Zellen, insbesondere Elektroporation und Lipofektion, verwendet werden. Enzymatische Reaktionen erfolgen gemäß Herstellerangaben oder in an sich bekannter Weise.

Ein Nukleinsäuremolekül oder eine Nukleinsäuresequenz betrifft erfindungsgemäß eine Nukleinsäure, die vorzugsweise Desoxyribonukleinsäure (DNA) oder Ribonukleinsäure (RNA) ist. Nukleinsäuren umfassen erfindungsgemäß genomische DNA, cDNA, mRNA, rekombinant hergestellte und chemisch synthetisierte Moleküle. Eine Nukleinsäure kann erfindungsgemäß als einzelsträngiges oder doppelsträngiges und lineares oder kovalent kreisförmig geschlossenes Molekül vorliegen.

"mRNA" bedeutet "messenger-RNA" und betrifft ein "Transkript", das unter Verwendung von DNA als Matrize produziert wird und selbst für ein Peptid oder Protein kodiert. Eine mRNA umfasst typischerweise eine 5'-nicht-translatierte Region, eine Protein-kodierende Region und eine 3'-nicht-translatierte Region. mRNA hat eine begrenzte Halbwertszeit in Zellen und in vitro. Erfindungsgemäß kann mRNA durch in vitro-Transkription von einer DNA-Matrize hergestellt werden. Zusätzlich zu den erfindungsgemäßen Modifikationen kann sie durch weitere stabilisierende Modifikationen und Capping modifiziert sein.

Weiterhin umfasst der Begriff "Nukleinsäure" auch eine chemische Derivatisierung einer Nukleinsäure an einer Nukleotidbase, am Zucker oder am Phosphat und Nukleinsäuren, die nicht in der Natur vorkommende Nukleotide und Nukleotidanaloga enthalten. Erfindungsgemäß betrifft eine "Nukleinsäuresequenz, die von einer Nukleinsäuresequenz abgeleitet ist" eine Nukleinsäure, in der im Vergleich zu der Nukleinsäure, von der sie abgeleitet ist, einzelne oder multiple Nukleotidsubstitutionen, -deletionen und/oder -additionen vorliegen und die zu der Nukleinsäure, von der sie abgeleitet ist, vorzugsweise komplementär ist, d.h. zwischen den Nukleinsäuren ist ein gewisser Grad an Homologie vorhanden und die Nukleinsäuren weisen in der Abfolge ihrer Nukleotide signifikante direkte oder komplementäre Übereinstimmungen auf. Eine von einer Nukleinsäure abgeleitete Nukleinsäure weist erfindungsgemäß eine funktionelle Eigenschaft der Nukleinsäure auf, von der sie abgeleitet ist. Solche funktionellen Eigenschaften umfassen insbesondere die Fähigkeit, bei einer funktionellen Verbindung mit einer Nukleinsäure, die in RNA transkribierbar ist (transkribierbare Nukleinsäuresequenz), die Stabilität und/oder die Translationseffizienz der von dieser Nukleinsäure gebildeten RNA in dem Gesamt-RNA-Molekül zu erhöhen.

"Funktionelle Verbindung" oder "funktionell verbunden" betrifft erfindungsgemäß eine Verbindung in einer funktionellen Beziehung. Eine Nukleinsäure ist "funktionell verbunden", wenn sie in eine funktionelle Beziehung mit einer anderen Nukleinsäuresequenz gesetzt wird. Zum Beispiel ist ein Promotor mit einer kodierenden Sequenz funktionell verbunden, falls er die Transkription der kodierenden Sequenz beeinflusst. Funktionell verbundene Nukleinsäuren sind typischerweise benachbart zueinander, gegebenenfalls getrennt durch weitere Nukleinsäuresequenzen, und werden in bestimmten Ausführungsformen in ein einzelnes RNA-Molekül (gemeinsames Transkript) durch RNA-Polymerase transkribiert.

Die erfindungsgemäß beschriebenen Nukleinsäuren sind vorzugsweise isoliert. Der Begriff "isolierte Nukleinsäure" bedeutet erfindungsgemäß, dass die Nukleinsäure (i) in vitro amplifiziert wurde, zum Beispiel durch Polymerase-Kettenreaktion (PCR), (ii) rekombinant durch Klonierung produziert wurde, (iii) gereinigt wurde, zum Beispiel durch Spaltung und gelelektrophoretische Auftrennung, oder (iv) synthetisiert wurde, zum Beispiel durch chemische Synthese. Eine isolierte Nukleinsäure ist eine Nukleinsäure, die für eine Manipulierung durch rekombinante DNA-Techniken zur Verfügung steht.

Eine Nukleinsäure ist dann zu einer anderen Nukleinsäure "komplementär", wenn die beiden Sequenzen miteinander hybridisieren und ein stabiles Duplex eingehen können, wobei die Hybridisierung vorzugsweise unter Bedingungen erfolgt, die eine spezifische Hybridisierung zwischen Polynukleotiden erlauben (stringente Bedingungen). Stringente Bedingungen sind beispielsweise in Molecular Cloning: A Laboratory Manual, J. Sambrook et al., Hrsg., 2. Auflage, Cold Spring Harbor Laboratory Press, Cold Spring Harbor, New York, 1989 oder Current Protocols in Molecular Biology, F.M. Ausubel et al., Hrsg., John Wiley & Sons, Inc., New York beschrieben und betreffen beispielsweise die Hybridisierung bei 65°C in Hybridisierungspuffer (3,5 x SSC, 0,02% Ficoll, 0,02% Polyvinylpyrrolidon, 0,02% Rinderserumalbumin, 2,5mM NaH₂PO₄ (pH7), 0,5% SDS, 2mM EDTA). SSC ist 0,15 M Natriumchlorid/ 0,15 M Natriumcitrat, pH 7. Nach der Hybridisierung wird die Membran, auf die die DNA übertragen wurde beispielsweise in 2 x SSC bei Raumtemperatur und sodann in 0,1 - 0,5 x SSC/ 0,1 x SDS bei Temperaturen bis 68°C gewaschen.

Komplementäre Nukleinsäuren weisen erfindungsgemäß mindestens 60%, mindestens 70%, mindestens 80%, mindestens 90% und vorzugsweise mindestens 95%, mindestens 98% oder mindestens 99% Identität der Nukleotide auf.

Der Begriff "% Identität" soll einen Prozentwert an Nukleotiden bezeichnen, die zwischen zwei zu vergleichenden Sequenzen bei einem optimalen Alignment identisch sind, wobei dieser Prozentwert rein statistisch ist, die Unterschiede zwischen den zwei Sequenzen zufällig und über die gesamte Sequenzlänge verteilt sein können und die zu vergleichende Sequenz Additionen oder Deletionen im Vergleich zu der Referenzsequenz umfassen kann, um ein optimales Alignment zwischen zwei Sequenzen zu erreichen. Sequenzvergleiche zwischen zwei Sequenzen werden im Allgemeinen durch Vergleich dieser Sequenzen nach einem optimalen Alignment in Bezug auf ein Segment oder "Vergleichsfenster" durchgeführt, um lokale Bereiche einer Sequenzübereinstimmung zu identifizieren. Das optimale Alignment für einen Vergleich kann manuell oder mit Hilfe des lokalen Homologiealgorithmus von Smith and Waterman, 1981, Ads App. Math. 2, 482, mit Hilfe des lokalen Homologiealgorithmus von Neddleman and Wunsch, 1970, J. Mol. Biol. 48,443, und mit Hilfe des Ähnlichkeitssuchalgorithmus von Pearson and Lipman, 1988, Proc. Natl Acad. Sci. USA 85, 2444, oder mit Hilfe von Computerprogrammen, die diese Algorithmen verwenden, (GAP, BESTFIT, FASTA, BLAST P, BLAST N and TFASTA in Wisconsin Genetics Software Package, Genetics Computer Group, 575 Science Drive, Madison, Wis.) durchgeführt werden.

Die prozentuale Identität wird durch Bestimmung der Anzahl an identischen Positionen, in denen die zu vergleichenden Sequenzen übereinstimmen, Teilung dieser Anzahl durch die verglichenen Positionen und Multiplikation dieses Ergebnisses mit 100 erhalten.

Beispielsweise kann das Programm BLAST "BLAST 2 sequences", das von der Website http://www.ncbi.nlm.nih.gov/blast/bl2seq/wblast2.cgi erhältlich ist, verwendet werden.

"3'-Ende einer Nukleinsäure" betrifft erfindungsgemäß dasjenige Ende, an dem sich eine freie Hydroxy-Gruppe befindet. In schematischer Darstellung von doppelsträngigen Nukleinsäuren, insbesondere DNA, befindet sich das 3'-Ende immer rechts. "5'-Ende einer Nukleinsäure" betrifft erfindungsgemäß dasjenige Ende, an dem sich eine freie Phosphat-Gruppe befindet. In schematischer Darstellung von doppelsträngigen Nukleinsäuren, insbesondere DNA, befindet sich das 5'-Ende immer links.

| | | |
|---|---|---|
| 5'-Ende | 5'--P-NNNNNNN-OH-3' | 3'-Ende |
| | 3'-HO-NNNNNNN-P--5' | |

In bestimmten Ausführungsformen liegt eine Nukleinsäure erfindungsgemäß funktionell in Verbindung mit Expressionskontrollsequenzen vor, die in Bezug zu der Nukleinsäure homolog oder heterolog sein können.

Eine transkribierbare Nukleinsäure, insbesondere eine für ein Peptid oder Protein kodierende Nukleinsäure, und eine Expressionskontrollsequenz sind dann "funktionell" miteinander verbunden, falls sie derart kovalent miteinander verknüpft sind, dass die Transkription oder Expression der transkribierbaren und insbesondere kodierenden Nukleinsäure unter der Kontrolle oder unter dem Einfluss der Expressionskontrollsequenz steht. Falls die Nukleinsäure in ein funktionelles Peptid oder Protein translatiert werden soll, führt bei einer funktionellen Verbindung einer Expressionskontrollsequenz mit der kodierenden Sequenz eine Induktion der Expressionskontrollsequenz zu einer Transkription der kodierenden Sequenz, ohne dass es zu einer Leserasterverschiebung in der kodierenden Sequenz oder zu einem Unvermögen der kodierenden Sequenz kommt, in das gewünschte Peptid oder Protein translatiert zu werden.

Der Begriff "Expressionskontrollsequenz" umfasst erfindungsgemäß Promotoren, ribosombindende Sequenzen und andere Kontrollelemente, welche die Transkription eines Gens oder die Translation der abgeleiteten RNA steuern. In bestimmten erfindungsgemäßen Ausführungsformen sind die Expressionskontrollsequenzen regulierbar. Die genaue Struktur von Expressionskontrollsequenzen kann speziesabhängig oder zelltypusabhängig variieren, umfasst jedoch im allgemeinen 5'-nicht-transkribierte und 5'- und 3'-nicht-translatierte Sequenzen, die an der Initiation der Transkription bzw. Translation beteiligt sind wie TATA-Box, Capping-Sequenz, CAAT-Sequenz und ähnliches. Insbesondere umfassen 5'-nicht-transkribierte-Expressionskontrollsequenzen eine Promotorregion, die eine Promotorsequenz für eine transkriptionelle Kontrolle des funktionell verbundenen Gens einschließt. Expressionskontrollsequenzen können auch Enhancer-Sequenzen oder stromaufwärts gelegene Aktivatorsequenzen umfassen.

Die hier dargestellten Nukleinsäuren, insbesondere transkribierbaren und kodierenden Nukleinsäuren, können mit beliebigen Expressionskontrollsequenzen, insbesondere Promotoren, kombiniert werden, die zu den Nukleinsäuren homolog oder heterolog sein können, wobei der Begriff "homolog" bezeichnet, dass eine Nukleinsäure auch natürlicherweise mit der Expressionskontrollsequenz funktionell verbunden ist und der Begriff "heterolog" bezeichnet, dass eine Nukleinsäure nicht natürlicherweise mit der Expressionskontrollsequenz funktionell verbunden ist.

Der Begriff "Promotor" oder "Promotorregion" betrifft eine DNA-Sequenz, die stromaufwärts (5') zu der kodierenden Sequenz eines Gens liegt und die Expression der kodierenden Sequenz durch Bereitstellen einer Erkennungs- und Bindungsstelle für RNA-Polymerase steuert. Die Promotorregion kann weitere Erkennungs- oder Bindungsstellen für weitere Faktoren beinhalten, die an der Regulation der Transkription des Gens beteiligt sind. Ein Promotor kann die Transkription eines prokaryontischen oder eukaryontischen Gens steuern. Ein Promotor kann "induzierbar" sein und die Transkription in Reaktion auf ein Induktionsmittel initiieren oder er kann "konstitutiv" sein, falls die Transkription nicht durch ein Induktionsmittel gesteuert wird. Ein induzierbarer Promotor wird nicht exprimiert oder nur in einem sehr geringen Maß exprimiert, wenn ein Induktionsmittel fehlt. In Gegenwart des Induktionsmittels wird das Gen "angeschalten" oder das Transkriptionsniveau erhöht. Dies wird herkömmlicherweise durch die Bindung eines spezifischen Transkriptionsfaktors vermittelt.

Erfindungsgemäß bevorzugte Promotoren sind beispielsweise Promotoren für SP6-, T3- oder T7-Polymerase.

Der Begriff "Expression" wird erfindungsgemäß in seiner allgemeinsten Bedeutung verwendet und umfasst die Produktion von RNA oder von RNA und Protein. Er umfasst auch eine teilweise Expression von Nukleinsäuren. Des weiteren kann die Expression transient oder stabil erfolgen.. In Bezug auf RNA betrifft der Begriff "Expression" oder "Translation" insbesondere die Produktion von Peptiden oder Proteinen.

Der Begriff "Nukleinsäuren, die in ein gemeinsames Transkript transkribierbar sind" bedeutet, dass die Nukleinsäuren derart funktionell miteinander verbunden sind, dass, gegebenenfalls nach Linearisierung wie Spaltung durch Restriktionsenzym des Nukleinsäuremoleküls, das die Nukleinsäuren umfasst, insbesondere eines kreisförmig geschlossenen Nukleinsäuremoleküls, eine Transkription unter Kontrolle eines Promotors in einem RNA-Molekül resultiert, das die Transkripte der Nukleinsäuren in kovalenter Bindung zueinander, gegebenenfalls durch dazwischenliegende Sequenzen getrennt, umfasst.

Der Begriff "Transkription" umfasst erfindungsgemäß "in vitro-Transkription", wobei der Begriff "in vitro-Transkription" ein Verfahren betrifft, bei dem RNA, insbesondere mRNA, zellfrei, d.h. vorzugsweise unter Verwendung entsprechend aufbereiteter Zellextrakte, in vitro synthetisiert wird. Vorzugsweise werden für die Herstellung von Transkripten Klonierungsvektoren eingesetzt, die im Allgemeinen als Transkriptionsvektoren bezeichnet werden und erfindungsgemäß von dem Begriff "Vektor" umfasst sind.

Der Begriff "von einer Nukleinsäuresequenz transkribierte Nukleinsäuresequenz" betrifft RNA, gegebenenfalls als Teil eines Gesamt-RNA-Moleküls, die ein Transkriptionsprodukt der ersten Nukleinsäuresequenz ist.

Der Begriff "Nukleinsäuresequenz, die wirksam ist, die Translationseffizienz und/oder die Stabilität einer Nukleinsäuresequenz zu erhöhen" bedeutet, dass die erste Nukleinsäure in der Lage ist, in einem gemeinsamen Transkript mit der zweiten Nukleinsäure die Translationseffizienz und/oder die Stabilität der zweiten Nukleinsäure derart zu modifizieren, dass diese im Vergleich zu der Translationseffizienz und/oder der Stabilität der zweiten Nukleinsäure ohne die erste Nukleinsäure gesteigert ist. Der Begriff "Translationseffizienz" betrifft in diesem Zusammenhang die in einem bestimmten Zeitraum von einem RNA-Molekül bereitgestellte Menge an Translationsprodukt und der Begriff "Stabilität" betrifft die Halbwertszeit eines RNA-Moleküls.

Die 3'-nicht-translatierte Region betrifft eine Region, die am 3'-Ende eines Gens stromabwärts zu dem Terminations-Codon einer Protein-kodierenden Region liegt, transkribiert wird, jedoch nicht in eine Aminosäuresequenz translatiert wird.

Erfindungsgemäß wird eine erste Polynukleotid-Region als zu einer zweiten Polynukleotid-Region stromabwärts gelegen betrachtet, wenn das 5'-Ende der ersten Polynukleotid-Region der zu dem 3'-Ende der zweiten Polynukleotid-Region nächstgelegene Teil der ersten Polynukleotid-Region ist.

Die 3'-nicht-translatierte Region erstreckt sich typischerweise von dem Terminations-Codon für ein Translationsprodukt bis zu der Poly(A)-Sequenz, die herkömmlicherweise nach dem Transkriptionsprozess angefügt wird. Die 3'-nicht-translatierten Regionen von Säuger-mRNA weisen typischerweise einen Homologiebereich auf, der als die AAUAAA-Hexanukleotidsequenz bekannt ist. Diese Sequenz ist vermutlich das Poly(A)-Anfügungssignal. Häufig liegt es 10 bis 30 Basen vor der Poly(A)-Anfügungsstelle.

3'-nicht-translatierte Regionen können eine oder mehrere invertierte Wiederholungen enthalten, die sich in Stamm-Schleife-Strukturen falten können, die als Barriere für Exoribonukleasen fungieren oder mit Proteinen interagieren, von denen bekannt ist, dass sie die RNA-Stabilität erhöhen (z.B. RNA-bindende Proteine).

5'- und/oder 3'-nicht-translatierte Regionen können erfindungsgemäß mit einer transkribierbaren und insbesondere kodierenden Nukleinsäure funktionell verbunden sein, so dass diese Regionen derart mit der Nukleinsäure in Beziehung steht, dass sie die Stabilität und/oder Translationseffizienz der von der transkribierbaren Nukleinsäure transkribierten RNA erhöht.

Die 3'-nicht-translatierten Regionen von Immunglobulin-mRNAs sind relativ kurz (weniger als etwa 300 Nukleotide), während die 3'-nicht-translatierten Regionen anderer Gene relativ lang sind. Zum Beispiel ist die 3'-nicht-translatierte Region von tPA etwa 800 Nukleotide lang, die von Faktor VIII ist etwa 1800 Nukleotide lang und die von Erythropoietin ist etwa 560 Nukleotide lang.

Erfindungsgemäß kann ermittelt werden, ob eine 3'-nicht-translatierte Region oder eine davon abgeleitete Nukleinsäuresequenz die Stabilität und/oder Translationseffizienz von RNA erhöht, indem die 3'-nicht-translatierte Region oder die davon abgeleitete Nukleinsäuresequenz in die 3'-nicht-translatierte Region eines Gens eingebaut wird und gemessen wird, ob dieser Einbau die Menge des synthetisierten Proteins erhöht.

Vorstehendes gilt sinngemäß für den Fall, dass erfindungsgemäß eine Nukleinsäure 2 oder mehrere 3'-nicht-translatierte Regionen umfasst, die vorzugsweise sequentiell mit oder ohne einen dazwischen liegenden Linker, vorzugsweise in einer "Kopf-an-Schwanz-Beziehung" (d.h. die 3'-nicht-translatierten Regionen weisen die gleiche Orientierung, vorzugsweise die in einer Nukleinsäure natürlich auftretende Orientierung auf) gekoppelt vorliegen.

Der Begriff "Gen" betrifft erfindungsgemäß eine bestimmte Nukleinsäuresequenz, die für die Produktion eines oder mehrerer zellulärer Produkte und/oder für das Erreichen einer oder mehrerer interzellulärer oder intrazellulärer Funktionen verantwortlich ist. Insbesondere betrifft der Begriff einen DNA-Abschnitt, welcher eine Nukleinsäure umfasst, die für ein spezifisches Protein oder ein funktionelles bzw. strukturelles RNA-Molekül kodiert.

Die Begriffe "Polyadenylkassette" oder "Poly(A)-Sequenz" betreffen eine Sequenz von Adenyl-Resten, die typischerweise am 3'-Ende eines RNA-Moleküls liegt. Erfindungsgemäß ist vorgesehen, dass eine solche Sequenz durch eine DNA-Matrize anhand von sich wiederholenden Thymidyl-Resten in dem zu dem kodierenden Strang komplementären Strang bei einer Transkription von RNA angefügt wird, wohingegen normalerweise sie nicht in der DNA kodiert ist, sondern durch eine matrizenunabhängige RNA-Polymerase nach der Transkription im Zellkern an das freie 3'-Ende der RNA angeheftet.wird. Erfindungsgemäß ist eine Nukleotidsequenz von mindestens 20, vorzugsweise mindestens 40, vorzugsweise mindestens 80, vorzugsweise mindestens 100 und vorzugsweise bis zu 500, vorzugsweise bis zu 400, vorzugsweise bis zu 300, vorzugsweise bis zu 200 und insbesondere bis zu 150 aufeinander folgenden A-Nukleotiden, und insbesondere etwa 120 aufeinander folgenden A-Nukleotiden als eine derartige Poly(A)-Sequenz zu verstehen, wobei der Begriff "A-Nukleotide" Adenyl-Reste bezeichnet.

In einer bevorzugten Ausführungsform ist ein Nukleinsäuremolekül erfindungsgemäß ein Vektor. Der Begriff "Vektor" wird dabei in seiner allgemeinsten Bedeutung verwendet und umfasst jegliche intermediären Vehikel für eine Nukleinsäure, die es z.B. ermöglichen, die Nukleinsäure in prokaryotische und/oder in eukaryotische Wirtszellen einzubringen und gegebenenfalls in ein Genom zu integrieren. Solche Vektoren werden vorzugsweise in der Zelle repliziert und/oder exprimiert. Vektoren umfassen Plasmide, Phagemide oder Virusgenome. Der Begriff "Plasmid", wie hierin verwendet, betrifft allgemein ein Konstrukt von extrachromosomalem genetischen Material, gewöhnlich ein zirkuläres DNA-Duplex, das unabhängig von chromosomaler DNA replizieren kann.

Der Begriff "Wirtszelle" betrifft erfindungsgemäß jede Zelle, die mit einer exogenen Nukleinsäure transformierbar oder transfizierbar ist. Der Begriff "Wirtszelle" umfasst erfindungsgemäß prokaryontische (z.B. E. coli) oder eukaryontische Zellen (z.B. Hefezellen und Insektenzellen). Besonders bevorzugt sind Säugerzellen wie Zellen aus Mensch, Maus, Hamster, Schwein, Ziege, Primaten. Die Zellen können aus einer Vielzahl von Gewebetypen abgeleitet sein und umfassen primäre Zellen und Zelllinien. Spezifische Beispiele umfassen Keratinozyten, periphere Blutleukozyten, Stammzellen des Knochenmarks und embryonale Stammzellen. In weiteren Ausführungsformen ist die Wirtszelle eine Antigen-präsentierende Zelle, insbesondere eine dendritische Zelle, ein Monozyt oder ein Makrophage. Eine Nukleinsäure kann in der Wirtszelle in einer einzigen oder in mehreren Kopien vorliegen und wird in einer Ausführungsform in der Wirtszelle exprimiert.

Erfindungsgemäß kann ein durch eine Nukleinsäure kodiertes Peptid oder Protein ein Peptid oder Protein sein, das im Cytoplasma, im Zellkern, in der Membran, in Organellen lokalisiert ist oder sekretiert wird. Sie beinhalten Strukturproteine, regulatorische Proteine, Hormone, Neurotransmitter, Wachstums-regulierende Faktoren, Differenzierungsfaktoren, Genexpressions-regulierende Faktoren, DNA-assoziierte Proteine, Enzyme, Serumproteine, Rezeptoren, Arzneimittel, Immunmodulatoren, Onkogene, Toxine, Tumorantigene oder Antigene. Diese Peptide oder Proteine können eine natürlich auftretende Sequenz oder eine mutierte Sequenz aufweisen, um ihre biologische Aktivität zu verstärken, inhibieren, regulieren oder zu beseitigen.

Der Begriff "Peptid" betrifft Substanzen, die zwei oder mehr, vorzugsweise 3 oder mehr, vorzugsweise 4 oder mehr, vorzugsweise 6 oder mehr, vorzugsweise 8 oder mehr, vorzugsweise 10 oder mehr, vorzugsweise 13 oder mehr, vorzugsweise 16 oder mehr, vorzugsweise 20 oder mehr and bis zu vorzugsweise 50, vorzugsweise 100 oder vorzugsweise 150 aufeinander folgende Aminosäuren umfassen, die durch Peptidbindungen miteinander verbunden sind. Der Begriff "Protein" betrifft große Peptide, vorzugsweise Peptide mit mindestens 151 Aminosäuren, jedoch werden die Begriffe "Peptid" und "Protein" hierin im Allgemeinen als Synonyme verwendet. Die Begriffe "Peptid" und "Protein" umfassen erfindungsgemäß Substanzen, die nicht nur Aminosäure-Bestandteile, sondern auch nicht-Aminosäure-Bestandteile wie Zucker und Phosphatstrukturen enthalten und umfassen auch Substanzen die Bindungen wie Ester-, Thioether- oder Disulfidbindungen enthalten.

Erfindungsgemäß vorgesehen ist eine Verabreichung von Nukleinsäuren, insbesondere RNA, an einen Patienten. In einer Ausführungsform erfolgt die Verabreichung von Nukleinsäuren durch ex vivo-Verfahren, d.h. durch Entfernung von Zellen aus einem Patienten, genetische Veränderung der Zellen, und Wiedereinbringung der veränderten Zellen in den Patienten. Transfektions- und Transduktionsverfahren sind dem Fachmann bekannt. Erfindungsgemäß vorgesehen ist auch eine Verabreichung von Nukleinsäuren in vivo.

Der Begriff "Transfektion" betrifft erfindungsgemäß das Einbringen einer oder mehrerer Nukleinsäuren in einen Organismus oder in eine Wirtszelle. Verschiedene Verfahren können eingesetzt werden, um erfindungsgemäß Nukleinsäuren in Zellen in vitro oder in vivo einzubringen. Solche Verfahren umfassen die Transfektion von Nukleinsäure-CaPO₄-Präzipitaten, die Transfektion von Nukleinsäuren, die mit DEAE assoziiert sind, die Transfektion oder Infektion mit Viren, die die interessierenden Nukleinsäuren tragen, die Liposomen-vermittelte Transfektion und ähnliches. In bestimmten Ausführungsformen ist eine Steuerung der Nukleinsäure an bestimmte Zellen bevorzugt. In solchen Ausführungsformen kann ein Träger, der für die Verabreichung einer Nukleinsäure an eine Zelle (z.B. ein Retrovirus oder ein Liposom) eingesetzt wird, ein gebundenes Zielsteuerungsmolekül aufweisen. Zum Beispiel kann ein Molekül wie ein Antikörper, der für ein Oberflächenmembran-Protein auf der Zielzelle spezifisch ist, oder ein Ligand für einen Rezeptor auf der Zielzelle in den Nukleinsäureträger eingebaut oder daran gebunden werden. Falls eine Verabreichung einer Nukleinsäure durch Liposomen erwünscht ist, können Proteine, die an ein Oberflächenmembran-Protein binden, das mit der Endozytose assoziiert ist, in die Liposomenformulierung eingebaut werden, um eine Zielsteuerung und/oder Aufnahme zu ermöglichen. Solche Proteine umfassen Kapsid-Proteine oder Fragmente davon, die für einen bestimmten Zelltyp spezifisch sind, Antikörper gegen Proteine, die internalisiert werden, Proteine, die eine intrazelluläre Stelle ansteuern, und ähnliches.

"Reporter" betrifft ein Molekül, typischerweise ein Peptid oder Protein, das durch ein Reporter-Gen kodiert wird und in einem Reportertest gemessen wird. Herkömmliche Systeme verwenden im Allgemeinen einen enzymatischen Reporter und messen die Aktivität des Reporters.

Der Begriff "multiple Klonierungsstelle" betrifft eine Nukleinsäureregion, die Restriktionsenzymstellen enthält, von denen irgendeine für eine Spaltung beispielsweise eines Vektors und die Insertion einer Nukleinsäure verwendet werden kann.

Zwei Elemente wie Nukleotide oder Aminosäuren sind erfindungsgemäß aufeinander folgend, falls sie ohne eine Unterbrechung unmittelbar zueinander benachbart sind. Beispielsweise betrifft eine Sequenz von x aufeinander folgenden Nukleotiden N die Sequenz (N)ₓ.

"Restriktions-Endonuklease" oder "Restriktionsenzym" bezeichnet eine Klasse von Enzymen, die in beiden Strängen eines DNA-Moleküls innerhalb spezifischer Basensequenzen Phosphodiester-Bindungen spalten. Sie erkennen auf einem doppelsträngigen DNA-Molekül spezifische Bindungsstellen, die als Erkennungsssequenzen bezeichnet werden. Die Stellen, an denen die Phosphodiester-Bindungen in der DNA durch die Enzyme gespalten werden, werden als Spaltstellen bezeichnet. Bei Typ IIS-Enzymen, liegt die Schnittstelle in einem definierten Abstand von der DNA-Bindungsstelle entfernt. Der Begriff "Restriktions-Endonuklease" umfasst erfindungsgemäß beispielsweise die Enzyme SapI, EciI, BpiI, AarI, AloI, BaeI, BbvCI, PpiI und PsrI, BsrD1, BtsI, EarI, BmrI, BsaI, BsmBI, FauI, BbsI, BciVI, BfuAI, BspMI, BseRI, EciI, BtgZI, BpuEI, BsgI, MmeI, CspCI, BaeI, BsaMI, Mva1269I, PctI, Bse3DI, BseMI, Bst6I, Eam1104I, Ksp632I, BfiI, Bso31I, BspTNI, Eco31I, Esp3I, BfuI, Acc36I, AarI, Eco57I, Eco57MI, GsuI, AloI, Hin4I, PpiI, und PsrI.

"Halbwertszeit" betrifft die Zeitspanne, die für eine Beseitigung der Hälfte der Aktivität, Menge oder Anzahl an Molekülen benötigt wird.

Die vorliegende Erfindung wird durch die nachstehenden Abbildungen und Beispiele ausführlich beschrieben, die ausschließlich der Erläuterung dienen und nicht begrenzend zu verstehen sind. Dem Fachmann sind aufgrund der Beschreibung und der Beispiele weitere Ausführungsformen zugänglich, die ebenfalls erfindungsgemäß umfasst sind.

### Abbildungen:

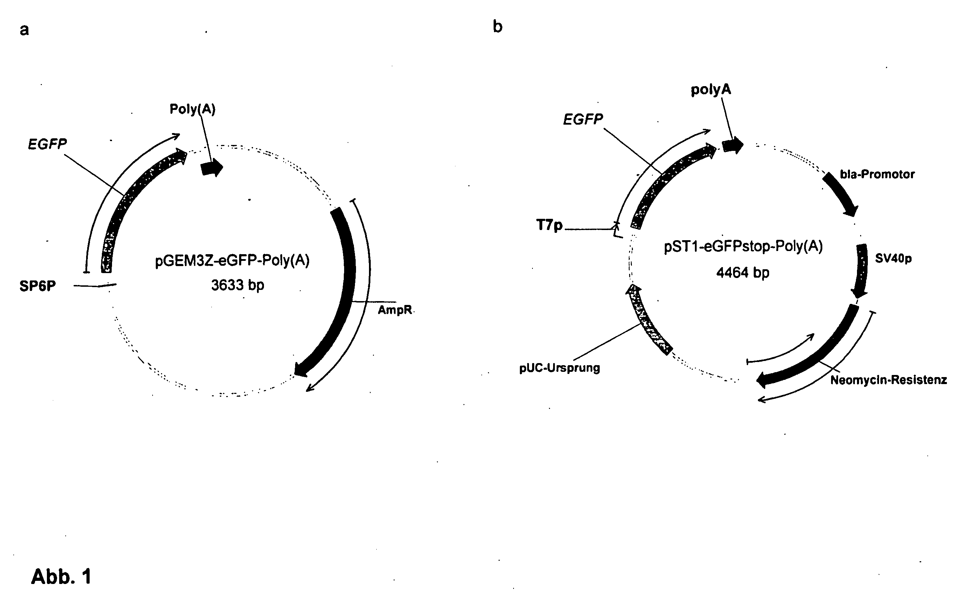

### Abb. 1: Erfindungsgemäß für die weitere Klonierung verwendete Basisvektoren

Die Vektoren erlauben eine Transkription von RNA unter der Kontrolle eines 5'-gelegenen RNA-Polymerase-Promoters und enthalten eine Polyadenylkassette.

### Abb. 2: Linearisierung von Vektoren mit Restriktionsenzymen vom Typ II (z.B SpeI) im Vergleich mit Restriktionsenzymen vom Typ IIS (z.B. SapI)

Durch die Einführung einer Restriktionsschnittstelle vom Typ IIS, deren Erkennungssequenz 3' von der Poly(A)-Sequenz liegt, während die Schnittstelle 24-26bp stromaufwärts und damit innerhalb der Poly(A)-Sequenz liegt, ist eine Linearisierung eines Plasmids innerhalb der Poly(A)-Sequenz möglich.

### Abb. 3: Vektoren, die erfindungsgemäß als Matrize für die in vitro Transkription hergestellt wurden

Um die Auswirkungen der erfindungsgemäßen RNA-Modifikationen auf Expressionshöhe und -dauer zu untersuchen, wurde eine Reihe von Vektoren hergestellt, die anschließend als Matrize für die in vitro-Transkription dienten. a. Vektoren mit verdeckter versus unverdeckter Poly(A)-Sequenz; b. Vektoren mit unterschiedlich langer Poly(A)-Sequenz; c. Vektoren mit 3'-nicht translatierter Region aus humanem Beta-Globin; d. SIINFEKL- und pp65-Vektoren; Cap - 5'-Capping; eGFP - GFP-Reportergen; 3'βg - 3'-nicht translatierte Region von β-Globin; A(x) - x bezeichnet die Anzahle der A-Nukleotide in der Poly(A)-Sequenz.

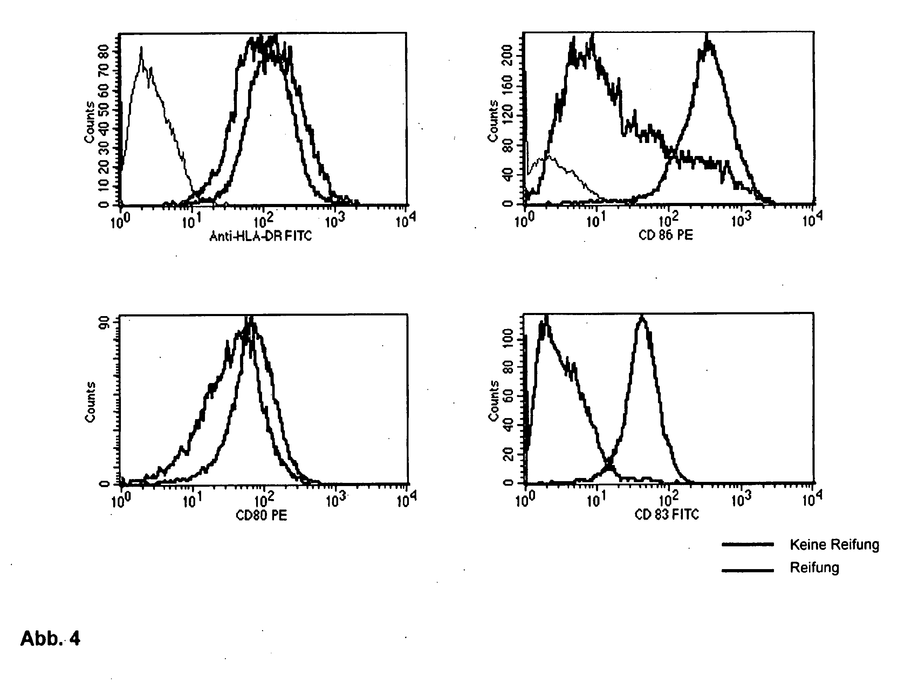

### Abb. 4: Bestimmung des Reifungsstatus von unreifen versus reifen Dendritischen Zellen über die angegebenen Oberflächenmarker

Die Wirkung der erfindungsgemäßen RNA-Modifikationen wurde in humanen Dendritischen Zellen (DC) gestestet, wobei bei einem immunogenen Reiz ein Reifungsprozess der DCs ausgelöst wird. Die DCs wurden mit anti-CD80-, anti-CD83-, anti-CD86- und anti-HLA-DR-Antikörpern, die spezifische DC-Reifungsmarker erkennen, gefärbt und durchflußzytometrisch analysiert.

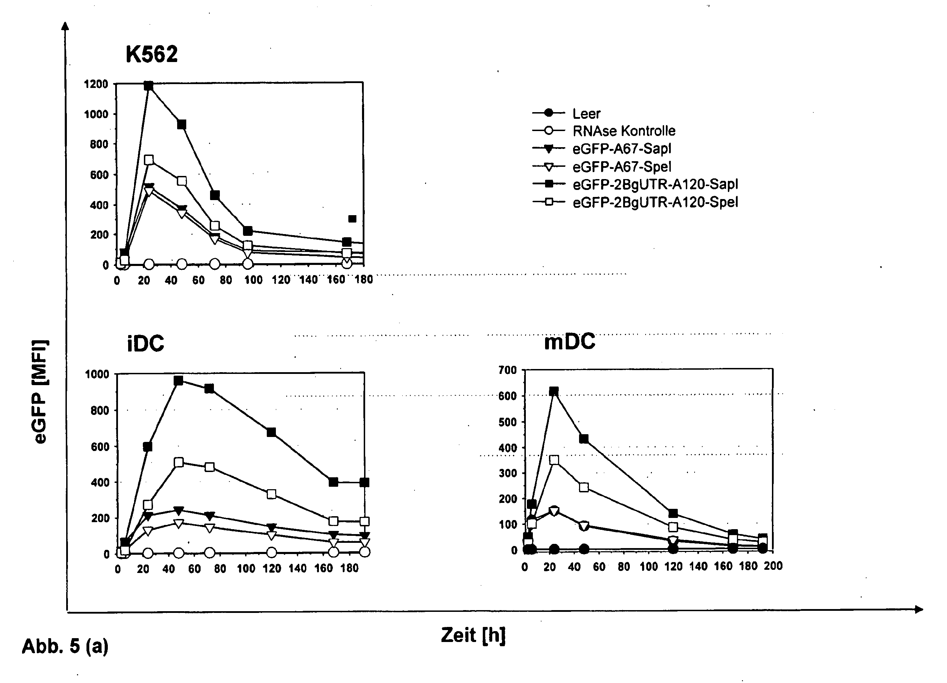

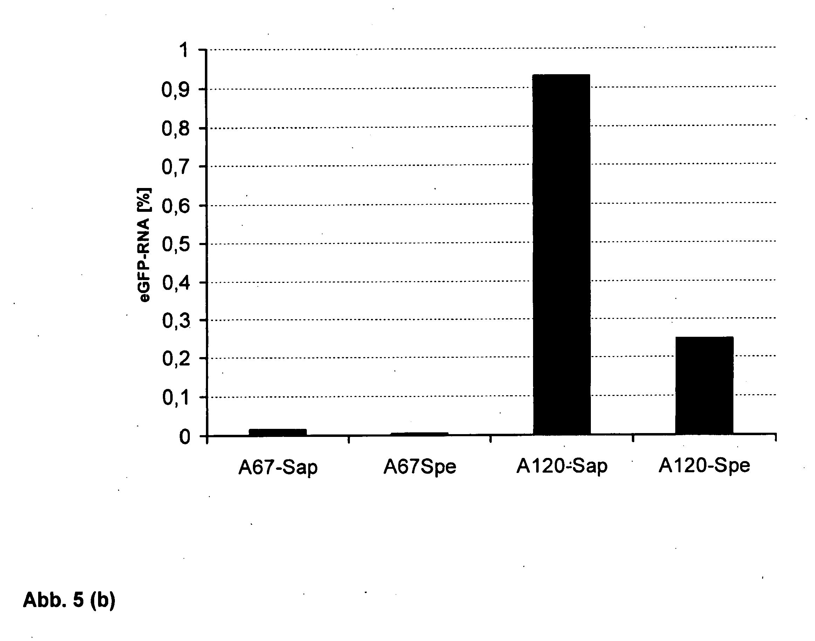

### Abb. 5: Einfluss von freier versus verdeckter Poly(A)-Sequenz auf die Translationseffizienz und Transkriptstabilität

a. Einfluss von freier versus verdeckter Poly(A)-Sequenz auf die Translationseffizienz von eGFP-RNA in K562- und Dendritischen Zellen durch die Ermittelung der mittleren Fluoreszenzintensität [MFI] im FACS-Kalibur; b. Einfluss von freier versus verdeckter Poly(A)-Sequenz auf die Transkriptstabilität von eGFP-RNA in unreifen Dendritischen Zellen nach 48h. Sowohl in der Tumorzellline als auch in unreifen DCs wird RNA mit offen endender Poly(A)-Sequenz effizienter und über einen längeren Zeitraum translatiert als RNA mit verdeckt endender Poly(A)-Sequenz. Bei gleicher Länge der Poly(A)-Sequenz ist die Translationseffizienz bei einer unverdeckt endenden Poly(A)-Sequenz in DCs um das 1,5-fache gesteigert. Eine offen endende Poly(A)-Sequenz führt außerdem zu einer höheren Stabilität der RNA.

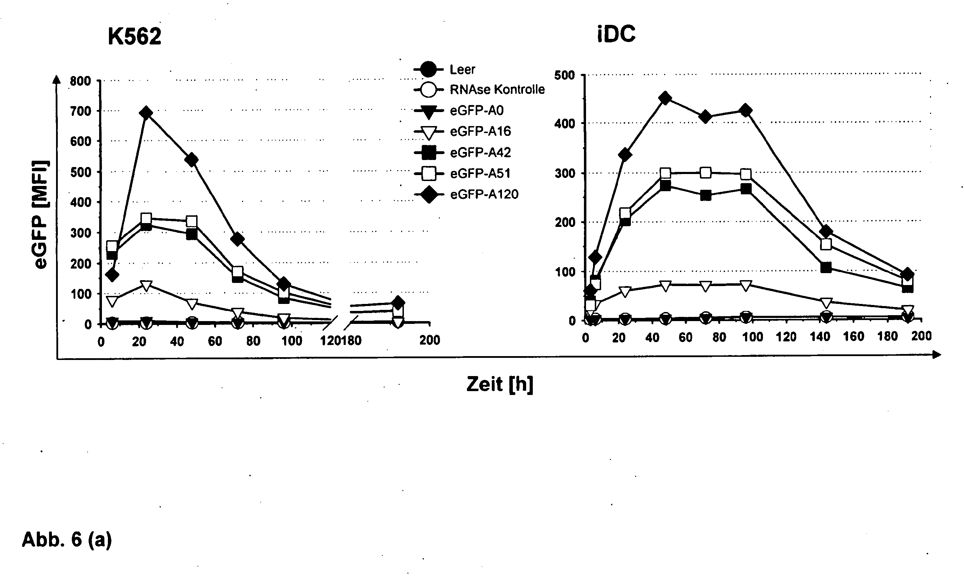

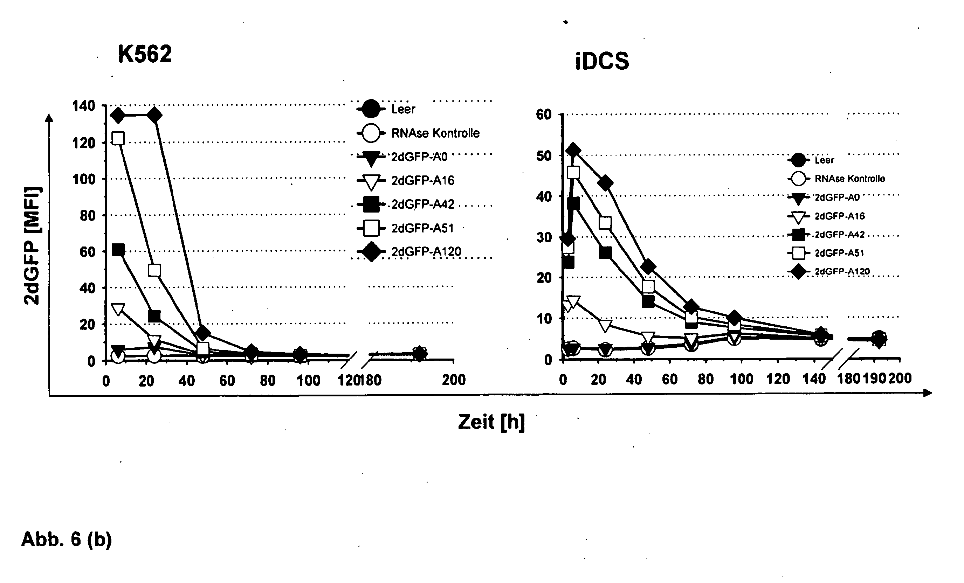

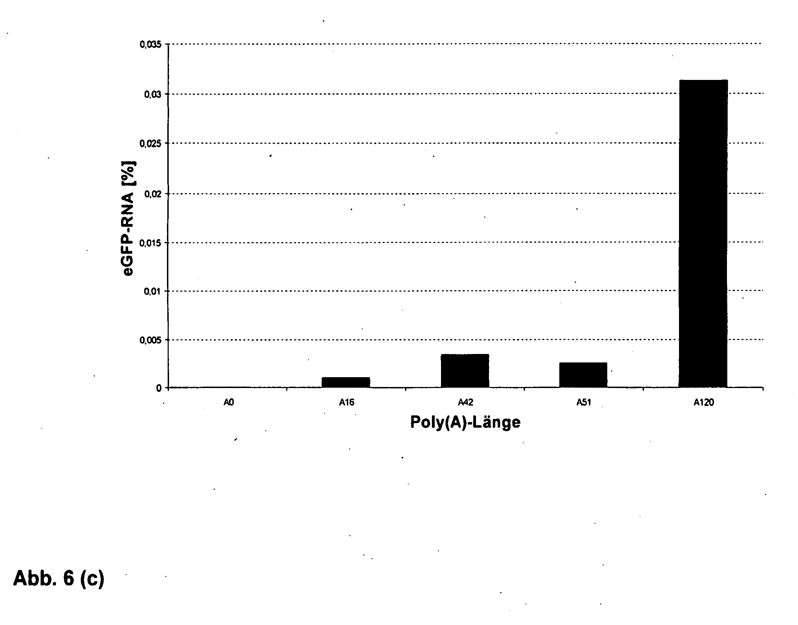

### Abb. 6: Einfluss der Länge der Poly(A)-Sequenz auf die Translationseffizienz und Transkriptstabilität

a. Einfluss der Länge der Poly(A)-Sequenz auf die Translationseffizienz von eGFP-RNA in K562₋ und Dendritischen Zellen; b. Einfluss der Länge der Poly(A)-Sequenz auf die Translationseffizienz von d2eGFP-RNA in K562- und Dendritischen Zellen; c. Einfluss der Länge der Poly(A)-Sequenz auf die Transkriptstabilität von eGFP-RNA in K562-Zellen 48h nach der Elektroporation. Eine Verlängerung der Poly(A)-Sequenz auf bis zu 120 A-Nukleotide führt zu einer Steigerung der Stabilität und Translation des Transkripts. Eine darüber hinausgehende Verlängerung hat keinen positiven Effekt. Durch die Verlängerung der Poly(A)-Sequenz von 51 auf 120 A-Nukleotide wird eine 1,5- bis 2-fache Zunahme der Translationseffizienz erreicht. Dieser Effekt spiegelt sich auch in der Stabilität der RNA.

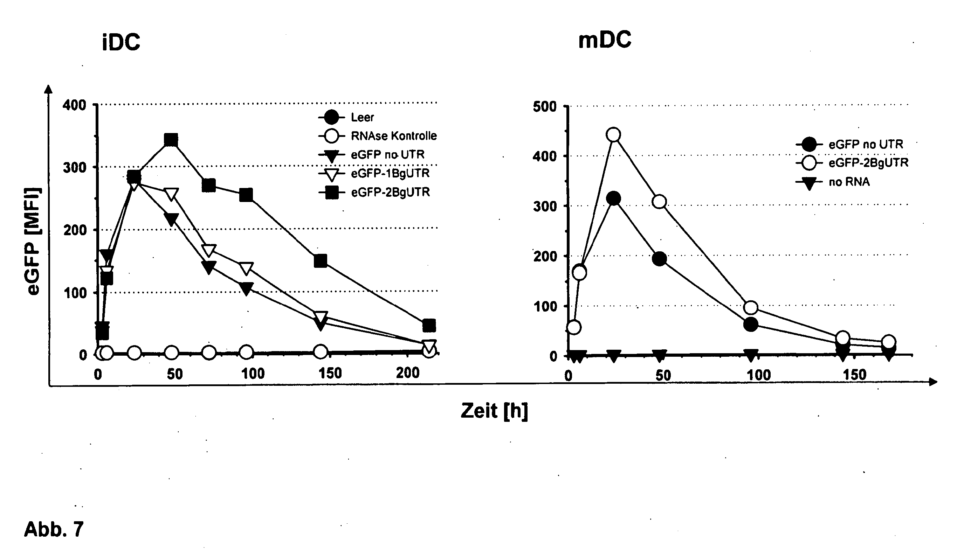

### Abb. 7: Einfluss einer 3'-nicht translatierten Region von humanem Beta-Globin (BgUTR) auf die Translationseffizienz in unreifen und reifen DCs

Ein Einfügen einer 3'-nicht translatierten Region von humanem Beta-Globin führt zu einer ansteigenden Expression des RNA-Transkripts. Eine doppelte 3'-nicht translatierten Region von humanem Beta-Globin führt nach 24h zu einer Verbesserung der Expressionshöhe, die über den Summationseffekt von zwei einzelnen 3'-nicht translatierten Region von humanem Beta-Globin deutlich hinausgeht.

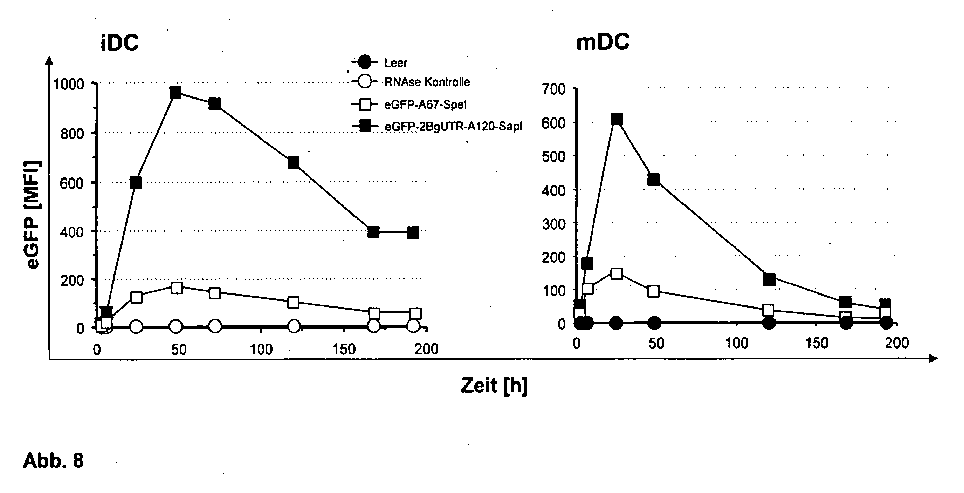

### Abb. 8: Wirkung der erfindungsgemäßen Modifikationen in Kombination auf die Translationseffizienz in unreifen und reifen DCs

Durch die Kombination der erfindungsgemäß beschriebenen Modifikationen eines RNA-Transkripts kann die Translationseffizienz von eGFP in unreifen und reifen DCs um mehr als das fünffache gesteigert werden.

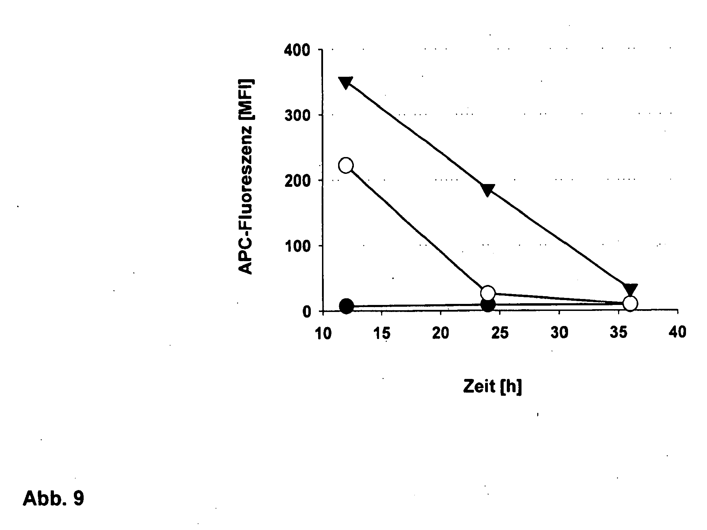

### Abb. 9: Wirkung der erfindungsgemäßen Modifikationen in Kombination auf die Präsentation von Peptiden durch MHC-Moleküle auf EL4-Zellen

Eine Verwendung der erfindungsgemäß modifizierten RNA-Konstrukte führt durch die gesteigerte Translationseffizienz zu einer verstärkten Präsentation von Peptid-MHC-Komplexen auf der Zelloberfläche. eGFP wurde in den beschriebenen IVT-Vektoren durch das OVA257-264-Epitop (SIINFEKL) ersetzt und als Zielzellen für die Transfektion EL4-Zellen (murin, T-Zell Lymphom) eingesetzt.

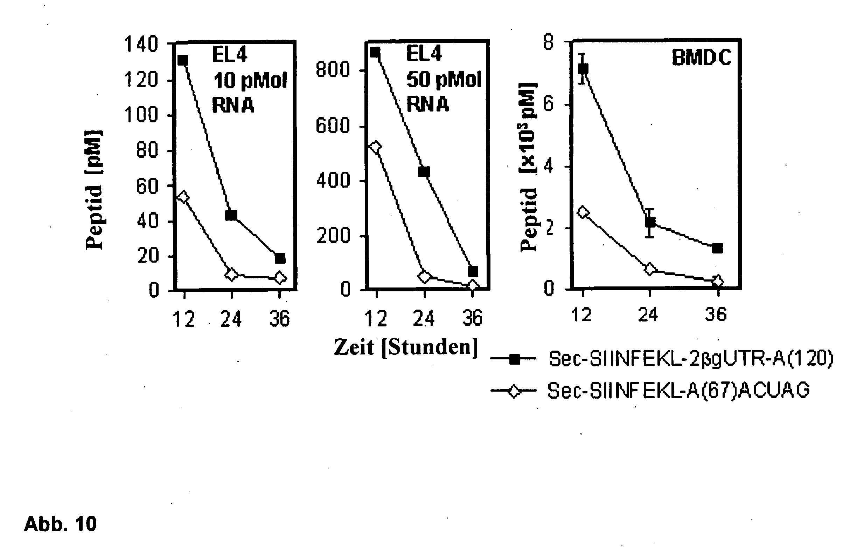

### Abb. 10: Erhöhung von Antigen-spezifischen Peptid/MHC-Komplexen durch Verwendung erfindungsgemäß stabilisierter IVT-RNA-Konstrukte

Zellen wurden mit Sec-SIINFEKL-A67-ACUAG-RNA oder Sec-SIINFEKL-2BgUTR-A120-RNA elektroporiert (EL4-Zellen: 10 pMol, 50 pMol; C57B1/J6-unreife BMDC in dreifachen Ansätzen: 150 pMol). Eine Elektroporation mit lediglich Puffer wurde als Kontrolle verwendet. Zellen wurden bezüglich SIINFEKL/K^{b}-Komplexen mit 25D1.16-Antikörper gefärbt. Konzentrationen von SIINFEKL-Peptid wurden aus den mittleren Fluoreszenzwerten von lebenden Zellen unter Verwendung einer Peptidtitration als Standardkurve berechnet. Daten für BMDC sind als Mittelwerte von drei Experimenten ± SEM gezeigt.

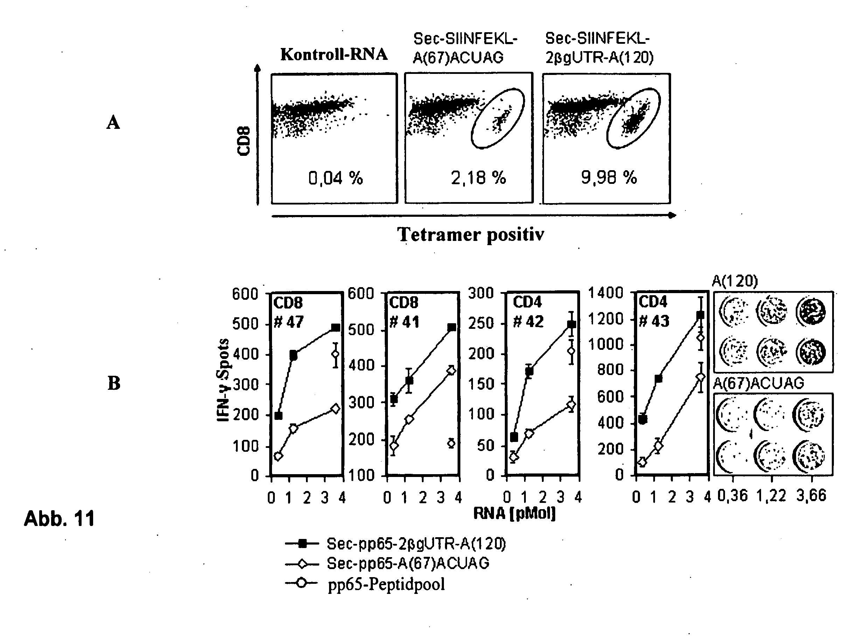

### Abb. 11: Wirkung von erfindungsgemäß stabilisierten IVT-RNA-Konstrukten auf die T-Zell-Stimulation in vivo und in vitro

(A) Verbesserte *in vivo*-T-Zell-Expansion durch Verwendung von stabilisierten IVT-RNA-Konstrukten. 1x10⁵-TCR-transgene CD8⁺ OT-I-Zellen wurden adoptiv in C57B1/J6-Mäuse transferiert. BMDC von C57B1/J6-Mäusen wurden mit 50 pMol RNA (Sec-SIINFEKL-A67-ACUAG-, Sec-SIINFEKL-2BgUTR-A120- oder Kontroll-RNA) transfiziert, 16h mit poly(I:C) (50 µg/ml) gereift und i.p. einen Tag nach T-Zell-Transfer injiziert (n=3). Peripheres Blut wurde am Tag 4 entnommen und bezüglich SIINFEKL-Tetramer-positiver CD8⁺-T-Zellen gefärbt. Dot-Blots zeigen CD8⁺-T-Zellen und die angegebenen Zahlen stellen den Prozentsatz an Tetramer-positiven CD8⁺-T-Zellen dar.
(B) Verbesserte *in vitro*-Expansion von humanen T-Zellen mit stabilisierten IVT-RNA-Konstrukten. CD8⁺ und CD4⁺ Lymphozyten aus HCMV-seropositiven gesunden Spendern wurden mit autologen DC co-kultiviert, die mit Sec-pp65-A67-ACUAG-RNA, Sec-pp65-2BgUTR-A120-RNA, oder Kontroll-RNA (Daten nicht gezeigt) transfiziert oder mit pp65-Peptidpool (1,75 µg/ml) als Positivkontrolle gepulst worden waren. Nach Expansion für 7 Tage wurde jede Effektorzellpopulation (4x10⁴/Well) in einem IFN-γ-ELISpot mit autologen DC (3x10⁴/Well), die entweder mit pp65-Peptidpool oder einem irrelevanten Peptidpool beladen worden waren (1,75 µg/ml), getestet. Die graphische Darstellung gibt die mittlere Anzahl an Spots von dreifachen Messungen ± SEM wieder.

### Beispiele:

### Beispiel 1: Herstellung von Vektoren und in vitro-Transkription von RNA

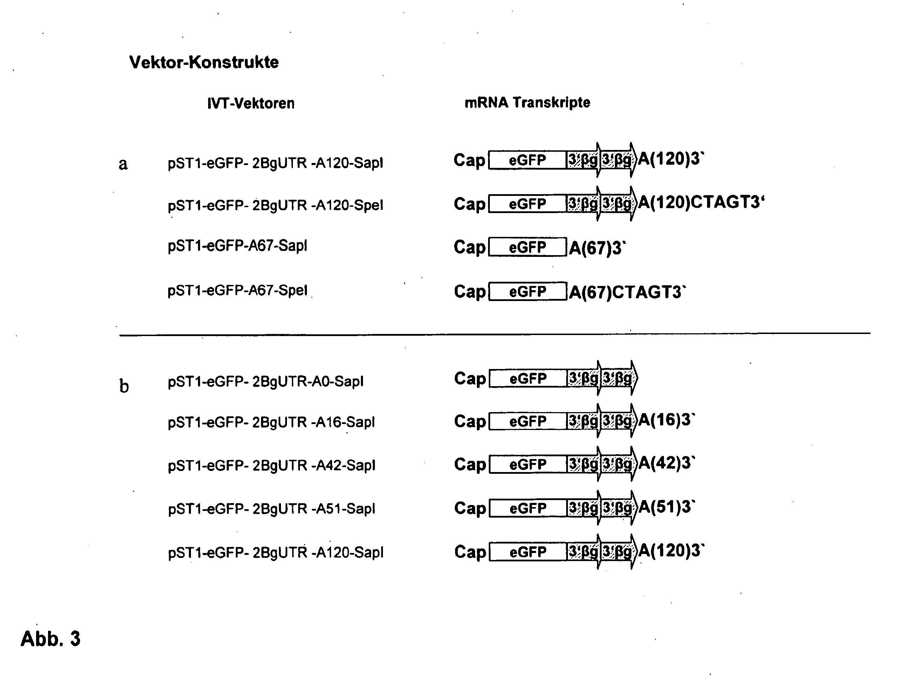

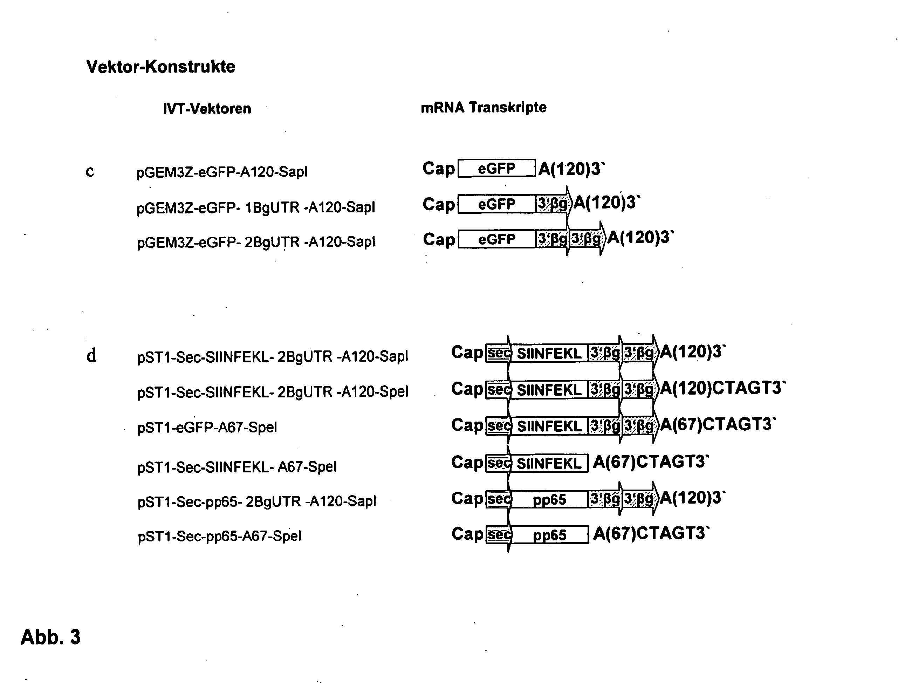

Um die Auswirkungen der erfindungsgemäßen RNA-Modifikationen auf Expressionshöhe und -dauer zu untersuchen, wurde eine Reihe von IVT-Vektoren hergestellt, die als Matrize für die in vitro-Transkription dienten (Abb. 3).

In die Vektoren wurden die Reportergene für eGFP bzw. d2eGFP, zwei Moleküle mit unterschiedlicher Halbwertszeit (HWZ), eingebracht, wodurch es möglich ist, den Einfluss der erfindungsgemäßen RNA-Modifikationen zu analysieren. Die Fluoreszenz fällt mit einer durchschnittlichen HWZ von 17,3 h bei eGFP und 2 h bei d2eGFP ab. Mit Hilfe dieser Konstrukte wurde in vitro-transkribierte eGFP-RNA bzw. d2eGFP-RNA hergestellt.

### Beispiel 2: Transfektion von Zellen mit der erfindungsgemäß modifizierten in vitro-transkribierten RNA und Wirkung auf die Translation und Stabilität der RNA

In vitro-transkribierte eGFP-RNA bzw. d2eGFP-RNA wurde dazu verwendet, K562-Zellen (human, Leukämie) mittels Elektroporation zu transfizieren. Die Transfektionseffizienz betrugt in K562 Zellen >90%.

Sodann wurde die Wirkung der beschriebenen RNA-Modifikationen in humanen Dendritischen Zellen (DC) gestestet, die die wichtigsten Modulatoren des Immunsystems darstellen. Dieser Ansatz ist von immunologischer Relevanz, da mit RNA transfizierte DCs für eine Vakzinierung in Betracht zu ziehen sind. Unreife DCs sind in der Haut und in peripheren Organen lokalisiert. Sie liegen hier in einem unreifen Zustand vor, der durch gut untersuchte Oberflächenmarker charakterisiert ist und sich funktionell durch eine starke Endozytoseaktivität auszeichnet. Bei einem immunogenen Reiz, wie z.B. einer Infektion mit Pathogenen, wird ein Reifungsprozess der DCs ausgelöst. Gleichzeitig wird ihre Wanderung in die regionalen Lymphknoten initiiert, wo sie die wirksamsten Induktoren von T-Zell- und B-Zell-Immunantworten sind. Auch der reife Zustand der DCs ist durch die Expression von detailliert untersuchten Oberflächenmarkem und Zytokinen sowie durch eine charakteristische Morphologie der DCs gekennzeichnet. Es gibt etablierte Zellkultursysteme zur Differenzierung von unreifen humanen DCs aus Blutmonozyten. Diese können durch verschiedene Stimuli zur Reifung gebracht werden.

In primären Dendritischen Zellen lag die Transfektionseffizienz bei 70-80%. Die DCs wurden mit anti-CD80-, anti-CD83-, anti-CD86- und anti-HLA-DR-Antikörpem, die spezifische DC-Reifungsmarker erkennen, gefärbt und durchflußzytometrisch analysiert (Abb. 4).

Die Bestimmung der Expressionshöhe und Dauer erfolgte mit Hilfe des FACS-Kalibur über die Ermittelung der Fluoreszenzintensität des eGFP. Die Ermittelung der RNA-Menge in den Zellen erfolgte mit Hilfe einer quantitativen RT-PCR.

### a. Wirkung einer offen endenden Poly(A)-Sequenz auf die Translation und Stabilität von RNA

Es konnte gezeigt werden, dass sowohl in der Tumorzellline K562 als auch in unreifen DCs (iDC) RNA mit offen endender Poly(A)-Sequenz effizienter und über einen längeren Zeitraum translatiert wird als RNA mit verdeckt endender Poly(A)-Sequenz (Abb. 5a). Bei gleicher Länge der Poly(A)-Sequenz ist die Translationseffizienz mit einer unverdeckt endenden Poly(A)-Sequenz in unreifen DCs um das 1,5-fache gesteigert. Die Modifikation führt außerdem zu einer höheren Stabilität der RNA (Abb. 5b). 48h nach der Elektroporation ist in unreifen DCs, die mit RNA mit einer unverdeckten poly(A)-Sequenz transifiziert worden waren, die 4- bis 5-fache Menge an RNA nachweisbar.

### b. Wirkung der Länge der Poly(A)-Sequenz auf die Translation und Stabilität von RNA

Durch Analyse von RNA mit Längen der Poly(A)-Sequenz von 16bp, 42bp, 51 bp, 67bp, 120bp, 200bp, 300bp und 600bp zeigte sich, dass die Verlängerung der Poly(A)-Sequenz auf bis zu 120 A-Nukleotide zu einer Steigerung der Transkriptstabilität und Translation führt und das eine darüber hinausgehende Verlängerung keinen positiven Effekt hat. Dieser Effekt zeigt sich sowohl in K562-Zellen als auch in unreifen DCs (iDC) (Abb. 6a und 6b). Durch die Verlängerung der Poly(A)-Sequenz von 51 auf 120 A-Nukleotide wird eine 1,5- bis 2-fache Zunahme der Translationseffizienz erreicht. Dieser Effekt spiegelt sich auch in der RNA-Stabilität (Abb. 6c).

### c. Wirkung des Auftretens einer 3'-nicht translatierten Region auf die Translation und Stabilität von RNA

In einer Zeitkinetik konnte mit K562-Zellen und unreifen DCs bestätigt werden, dass das Einfügen einer 3'-nicht translatierten Region (UTR) aus humanem Beta-Globin zu einer ansteigenden Expression eines RNA-Transkript führt. Darüber hinaus konnte gezeigt werden, dass eine doppelte 3'-nicht translatierten Region (UTR) aus humanem Beta-Globin nach 24h zu einer Verbesserung der Expressionshöhe führt, die über den Summationseffekt von zwei einzelnen UTRs deutlich hinausgeht (Abb. 7).

### d. Wirkung einer Kombination der vorstehend beschriebenen Modifikationen auf die Translation und Stabilität von RNA

Erfindungsgemäß konnte gezeigt werden, dass durch eine Kombination der vorstehend beschriebenen Modifikationen in einem RNA-Transkript die Translationseffizienz von eGFP in unreifen als auch reifen DCs um mehr als das fünffache gesteigert wird (Abb. 8).

### Beispiel 3: Präsentation eines durch in vitro-transkribierte RNA mit erhöhter Stabilität und Translationseffizienz exprimierten Peptids durch MHC-Moleküle

Es konnte erfindungsgemäß gezeigt werden, dass die Verwendung von erfindungsgemäß modifizierten RNA-Konstrukten zu einer erhöhten Peptid-MHC-Präsentation auf der Zelloberfläche führt. Hierfür wurde die für das eGFP kodierende Nukleinsäuresequenz in den beschriebenen IVT-Vektoren durch eine für das OVA257-264-Epitop (SIINFEKL) kodierende Nukleinsäuresequenz ersetzt und die Konstrukte miteinander verglichen. Als Zielzellen für die Transfektion wurden EL4-Zellen (murin, T-Zell Lymphom) eingesetzt.

Um durch MHC-Moleküle präsentierte SIINFEKL-Peptide zu quantifizieren, wurden die Zellen zu verschiedenen Zeitpunkten nach der Elektroporation mit einem anti-H2-K^{b}-OVA257-264-Antikörper gefärbt und die Fluoreszenzintensität eines sekundären Antikörpers mit Hilfe des FACS-Kalibur bestimmt (Abb. 9).

Des weiteren wurde das SIINFEKL-Peptid in den Vektor, der alle Optimierungen widerspiegelte (pST1-Sec-SIIFEKL-2BgUTR-A120-Sap1), und in einen Vektor mit Standardmerkmalen (pST1-Sec-SIINFEKL-A67-Spe1) kloniert. IVT-RNA, die von beiden Vektoren abgeleitet war, wurde in EL4-Zellen und BMDC elektroporiert. Signifikant höhere Zahlen an OVA-Peptid/K^{b}-Komplexen auf der Zelloberfläche wurden festgestellt, die über einen längeren zeitlichen Verlauf nach Elektroporation der erfindungsgemäß modifizierten RNA, Sec-SIINFEKL-2 BgUTR-A120, aufrechterhalten wurden (Abb. 10).

### Beispiel 4: Wirkung einer.Transfektion von Zellen mit in vitro-transkribierter RNA, die für eine zu präsentierendes Peptid kodiert, auf die Expansion von Antigen-spezifischen T-Zellen

Um die Wirkung auf die stimulatorische Kapazität zu bewerten, wurde auf den OT-I-TCR zurückgegriffen, der intensiv in dem C57BL/J6 (B6)-Hintergrund verwendet worden war, um MHC Klasse I-Präsentation des SIINFEKL-Peptids nachzuweisen. OT-1 CD8⁺-T-Zellen, die bezüglich des T-Zellrezeptors (TCR) transgen sind und das K^{b}-spezifische Peptid SIINFEKL aus Hühner-OVA (OVA₂₅₇₋₂₆₄) erkennen, wurden freundlicherweise von H. Schild (Institut für Immunologie, Universität Mainz) bereitgestellt.

Am Tag 0 wurden Tiere adoptiv mit OT-I-CD8⁺-T-Zellen transferiert. Hierzu wurden Splenozyten aus TCR tg OT-I-Mäusen hergestellt und in die Schwanzvene von C57BL/J6-Empfängermäusen eingebracht. Die Zellzahl wurde auf 1x10⁵ TCR tg CD8⁺-T-Zellen eingestellt. Am nächsten Tag wurden 1x10⁶ BMDC von C57BL/J6-Mäusen, die mit 50 pMol SIINFEKL-kodierenden RNA-Konstruktvarianten elektroporiert und 16 Stunden mittels Poly(I:C) gereift worden waren, i.p. an Mäuse verabreicht. Am Tag 4 wurden OT-I-CD8⁺-T-Zellen in peripherem Blut mit Hilfe der Tetramer-Technologie gemessen. Hierzu wurden retroorbitale Blutproben entnommen und mit Anti-CD8 (Caltag Laboratories, Burlingame, USA) und SIINFEKL-Tetramer (H-2Kb/SIINFEKL 257-264; Beckman Coulter, Fullerton, USA) gefärbt.

Es stellte sich heraus, dass eine *in vivo*-Expansion von Antigen-spezifischen TCR-transgenen CD8⁺-T-Zellen bei Verwendung von Sec-SIINFEKL-2BgUTR-A120-RNA für eine Antigenzufuhr signifikant besser im Vergleich zu Sec-SIINFEKL-A67-ACUAG-RNA war (Abb. 11A).

Um zu bewerten, ob stabilisierte IVT-RNA-Konstrukte für eine Antigenzufuhr auch eine Antigen-spezifische Stimulierung von humanen T-Zellen verbessern, wurde auf HCMV-pp65 zurückgegriffen, das immunodominante Antigen von humanem Cytomegalovirus, das häufig für die Validierung einer autologen Stimulation von polyepitopischen T-Zell-Reaktionen verwendet wird. CD4⁺- und CD8⁺-T-Zellen, die aus HCMV-seropositiven gesunden Spendern durch positive magnetische Zellsortierung mittels Antikörper-beschichteten Microbeads (Miltenyi Biotec, Bergisch-Gladbach, Deutschland) aufgereinigt worden waren, wurden mit 2x10⁵ autologen DC co-kultiviert, die mit den entsprechenden IVT-RNA-Varianten, die für pp65 kodieren, elektroporiert worden waren. Eine Expansion von T-Zellen, gemessen am Tag 7 in einem IFN-γ-ELISpot unter Verwendung von autologen DC, die mit einem Pool von überlappenden Peptiden, die die gesamte pp65-Proteinsequenz abdecken, oder einem Kontrollprotein gepulst worden waren, zeigte die Überlegenheit von Sec-pp65-2BgUTR-A 120, wobei die Effekte bezüglich einer Expansion von CD4⁺-T-Zellen am ausgeprägtesten waren (Abb. 11B).

### Literaturverzeichnis:

Bargmann,C.I., Hung,M.C., and Weinberg,R.A. (1986). The neu oncogene encodes an epidermal growth factor receptor-related protein. Nature 319, 226-230.
Boczkowski,D., Nair,S.K., Nam,J.H., Lyerly,H.K., and Gilboa,E. (2000). Induction of tumor immunity and cytotoxic T lymphocyte responses using dendritic cells transfected with messenger RNA amplified from tumor cells. Cancer Res. 60, 1028-1034.
Carralot,J.P., Probst,J., Hoerr,I., Scheel,B., Teufel,R., Jung,G., Rammensee,H.G., and Pascolo,S. (2004). Polarization of immunity induced by direct injection of naked sequencestabilized mRNA vaccines. Cell Mol. Life Sci. 61, 2418-2424.
Condon,C., Watkins,S.C., Celluzzi,C.M., Thompson,K., and Falo,L.D., Jr. (1996). DNA-based immunization by in vivo transfection of dendritic cells. Nat. Med. 2, 1122-1128.
Conry,R.M., LoBuglio,A.F., Kantor,J., Schlom,J., Loechel,F., Moore,S.E., Sumerel,L.A., Barlow,D.L., Abrams,S., and Curiel,D.T. (1994). Immune response to a carcinoembryonic antigen polynucleotide vaccine. Cancer Res. 54, 1164-1168.
Conry,R.M., LoBuglio,A.F., Loechel,F., Moore,S.E., Sumerel,L.A., Barlow,D.L., and Curiel,D.T. (1995a). A carcinoembryonic antigen polynucleotide vaccine has in vivo antitumor activity. Gene Ther. 2, 59-65.
Conry,R.M., LoBuglio,A.F., Wright,M., Sumerel,L., Pike,M.J., Johanning,F., Benjamin,R., Lu,D., and Curiel,D.T. (1995b). Characterization of a messenger RNA polynucleotide vaccine vector. Cancer Res. 55, 1397-1400.
Cox,G.J., Zamb,T.J., and Babiuk,L.A. (1993). Bovine herpesvirus 1: immune responses in mice and cattle injected with plasmid DNA. J. Virol. 67, 5664-5667.
Davis,H.L., Michel,M.L., and Whalen,R.G. (1993). DNA-based immunization induces continuous secretion of hepatitis B surface antigen and high levels of circulating antibody. Hum. Mol. Genet. 2, 1847-1851.
Gallie,D.R. (1991). The cap and poly(A) tail function synergistically to regulate mRNA translational efficiency. Genes Dev. 5, 2108-2116.
Gilkeson,G.S., Pippen,A.M., and Pisetsky,D.S. (1995). Induction of cross-reactive antidsDNA antibodies in preautoimmune NZB/NZW mice by immunization with bacterial DNA. J. Clin. Invest 95, 1398-1402.
Greenblatt,M.S., Bennett,W.P., Hollstein,M., and Harris,C.C. (1994). Mutations in the p53 tumor suppressor gene: clues to cancer etiology and molecular pathogenesis. Cancer Res. 54, 4855-4878.
Heiser,A., Coleman,D., Dannull,J., Yancey,D., Maurice,M.A., Lallas,C.D., Dahm,P., Niedzwiecki,D., Gilboa,E., and Vieweg,J. (2002). Autologous dendritic cells transfected with prostate-specific antigen RNA stimulate CTL responses against metastatic prostate tumors. J. Clin. Invest 109, 409-417.
Heiser,A., Dahm,P., Yancey,D.R., Maurice,M.A., Boczkowski,D., Nair,S.K., Gilboa,E., and Vieweg,J. (2000). Human dendritic cells transfected with RNA encoding prostate-specific antigen stimulate prostate-specific CTL responses in vitro. J. Immunol. 164, 5508-5514.
Hoerr,I., Obst,R., Rammensee,H.G., and Jung,G. (2000). In vivo application of RNA leads to induction of specific cytotoxic T lymphocytes and antibodies. Eur. J. Immunol. 30, 1-7.
Malone,R.W., Felgner,P.L., and Verma,I.M. (1989). Cationic liposome-mediated RNA transfection. Proc. Natl. Acad. Sci. U.S. A 86, 6077-6081.
Preiss,T. and Hentze,M.W. (1998). Dual function of the messenger RNA cap structure in poly(A)-tail-promoted translation in yeast. Nature 392, 516-520.
Spooner,R.A., Deonarain,M.P., and Epenetos,A.A. (1995). DNA vaccination for cancer treatment. Gene Ther. 2, 173-180.
Strong,T.V., Hampton,T.A., Louro,I., Bilbao,G., Conry,R.M., and Curiel,D.T. (1997). Incorporation of beta-globin untranslated regions into a Sindbis virus vector for augmentation of heterologous mRNA expression. Gene Ther. 4, 624-627.
Su,Z., Dannull,J., Heiser,A., Yancey,D., Pruitt,S., Madden,J., Coleman,D., Niedzwiecki,D., Gilboa,E., and Vieweg,J. (2003). Immunological and clinical responses in metastatic renal cancer patients vaccinated with tumor RNA-transfected dendritic cells. Cancer Res. 63, 2127-2133.
Tang,D.C., DeVit,M., and Johnston,S.A. (1992). Genetic immunization is a simple method for eliciting an immune response. Nature 356, 152-154.
Teufel,R., Carralot,J.P., Scheel,B., Probst,J., Walter,S., Jung,G., Hoerr,I., Rammensee,H.G., and Pascolo,S. (2005). Human peripheral blood monuclear cells transfected with messenger RNA stimulate antigen-specific cytotoxic T-lymphocytes in vitro. Cell Mol. Life Sci. 62, 1755-1762.
Ulmer,J.B., Donnelly,J.J., Parker,S.E., Rhodes,G.H., Felgner,P.L., Dwarki,V.J., Gromkowski,S.H., Deck,R.R., DeWitt,C.M., Friedman,A., and. (1993). Heterologous protection against influenza by injection of DNA encoding a viral protein. Science 259, 1745-1749.
Wang,B., Merva,M., Dang,K., Ugen,K.E., Williams,W.V., and Weiner,D.B. (1995). Immunization by direct DNA inoculation induces rejection of tumor cell challenge. Hum. Gene Ther. 6,407-418.
Wang,B., Ugen,K.E., Srikantan,V., Agadjanyan,M.G., Dang,K., Refaeli,Y., Sato,A.I., Boyer,J., Williams,W.V., and Weiner,D.B. (1993). Gene inoculation generates immune responses against human immunodeficiency virus type 1. Proc. Natl. Acad. Sci. U. S. A 90, 4156-4160.
Wolff,J.A., Malone,R.W., Williams,P., Chong,W., Acsadi,G., Jani,A., and Felgner,P.L. (1990). Direct gene transfer into mouse muscle in vivo. Science 247, 1465-1468.
Ying,H., Zaks,T.Z., Wang,R.F., Irvine,K.R., Kammula,U.S., Marincola,F.M., Leitner,W.W., and Restifo,N.P. (1999). Cancer therapy using a self-replicating RNA vaccine. Nat. Med. 5, 823-827.

## Patentansprüche

1. Nukleinsäuremolekül, umfassend in 5'→3'-Richtung der Transkription:
(a) einen Promotor,
(b) eine transkribierbare Nukleinsäuresequenz oder eine Nukleinsäuresequenz für ein Einbringen einer transkribierbaren Nukleinsäuresequenz,
(c-1) eine erste Nukleinsäuresequenz,
(c-2) eine zweite Nukleinsäuresequenz, und gegebenenfalls
(c-3) mindestens eine weitere Nukleinsäuresequenz,
wobei die Nukleinsäuresequenzen (c-1), (c-2) und gegebenenfalls (c-3) ausgewählt sind aus der Gruppe bestehend aus:
(I) einer Nukleinsäuresequenz, die der 3'-nicht-translatierten Region eines Globin-Gens entspricht, und
(II) einer Nukleinsäuresequenz, die zu der Nukleinsäuresequenz unter (I) mindestens 90% Identität aufweist,
wobei die Nukleinsäuresequenzen (c-1), (c-2) und gegebenenfalls (c-3) unabhängig voneinander von einem Gen abgeleitet sind, ausgewählt aus der Gruppe, bestehend aus alpha2-Globin-Gen, alphal-Globin-Gen, und beta-Globin-Gen, und
wobei die Nukleinsäuresequenzen (b), (c-1), (c-2) und gegebenenfalls (c-3) unter Kontrolle des Promotors (a) in ein gemeinsames Transkript transkribierbar sind und in dem gemeinsamen Transkript die von den Nukleinsäuresequenzen (c-1), (c-2) und gegebenenfalls (c-3) transkribierten Nukleinsäuresequenzen wirksam sind,
die Translationseffizienz und/oder die Stabilität der von der transkribierbaren Nukleinsäuresequenz (b) transkribierten Nukleinsäuresequenz zu erhöhen.

2. Nukleinsäuremolekül nach Anspruch 1, wobei die Nukleinsäuresequenzen (c-1), (c-2) und gegebenenfalls (c-3) gleich oder verschieden sein können.

3. Nukleinsäuremolekül nach Anspruch 1 oder 2, das ferner (d) eine Nukleinsäuresequenz umfasst, die bei einer Transkription unter Kontrolle des Promotors (a) für eine Nukleotidsequenz von mindestens 20 aufeinander folgenden A-Nukleotiden in dem Transkript kodiert.

4. Nukleinsäuremolekül nach Anspruch 3, wobei die Nukleinsäuresequenzen (b), (c-1), (c-2), gegebenenfalls (c-3), und (d) unter Kontrolle des Promotors (a) in ein gemeinsames Transkript transkribierbar sind und in dem gemeinsamen Transkript die von den Nukleinsäuresequenzen (c-1), (c-2), gegebenenfalls (c-3) und (d) transkribierten Nukleinsäuresequenzen wirksam sind, die Translationseffizienz und/oder die Stabilität der von der transkribierbaren Nukleinsäuresequenz (b) transkribierten Nukleinsäuresequenz zu erhöhen.

5. Nukleinsäuremolekül nach Anspruch 3 oder 4, wobei die Nukleinsäuresequenz (d) bei einer Transkription unter Kontrolle des Promotors (a) für eine Nukleotidsequenz von mindestens 40 aufeinander folgenden A-Nukleotiden in dem Transkript kodiert.

6. Nukleinsäuremolekül nach Anspruch 5, wobei die Nukleinsäuresequenz (d) bei einer Transkription unter Kontrolle des Promotors (a) für eine Nukleotidsequenz von mindestens 80 aufeinander folgenden A-Nukleotiden in dem Transkript kodiert.

7. Nukleinsäuremolekül nach Anspruch 6, wobei die Nukleinsäuresequenz (d) bei einer Transkription unter Kontrolle des Promotors (a) für eine Nukleotidsequenz von mindestens 100 aufeinander folgenden A-Nukleotiden in dem Transkript kodiert.

8. Nukleinsäuremolekül nach Anspruch 7, wobei die Nukleinsäuresequenz (d) bei einer Transkription unter Kontrolle des Promotors (a) für eine Nukleotidsequenz von etwa 120 aufeinander folgenden A-Nukleotiden in dem Transkript kodiert.

9. Nukleinsäuremolekül nach einem der Ansprüche 3 bis 8, **dadurch gekennzeichnet, dass** es innerhalb der Nukleinsäuresequenz (d) vorzugsweise enzymatisch oder anderweitig biochemisch derart spaltbar ist, dass durch die Spaltung ein Nukleinsäuremolekül entsteht, das in 5'→3'-Richtung der Transkription den Promotor (a), die Nukleinsäuresequenz (b), die Nukleinsäuresequenzen (c-1), (c-2), gegebenenfalls (c-3), und zumindest einen Teil der Nukleinsäuresequenz (d) umfasst, wobei der zumindest eine Teil der Nukleinsäuresequenz (d) bei einer Transkription unter Kontrolle des Promotors (a) für eine Nukleotidsequenz von mindestens 20 aufeinander folgenden A-Nukleotiden in dem Transkript kodiert und das Transkript als 3'-terminales Nukleotid ein A-Nukleotid aus der Nukleotidsequenz von mindestens 20 aufeinander folgenden A-Nukleotiden aufweist.

10. Nukleinsäuremolekül nach Anspruch 9, wobei das Nukleinsäuremolekül nach der Spaltung am Ende des Stranges, der als Matrize für die Nukleotidsequenz von mindestens 20 aufeinander folgenden A-Nukleotiden dient, ein T-Nukleotid aufweist, das Teil der Nukleotidsequenz ist, die als Matrize für die Nukleotidsequenz von mindestens 20 aufeinander folgenden A-Nukleotiden in dem Transkript dient.

11. Nukleinsäuremolekül nach Anspruch 9 oder 10, wobei der zumindest eine Teil der Nukleinsäuresequenz (d) bei einer Transkription unter Kontrolle des Promotors (a) für eine Nukleotidsequenz von mindestens 40 aufeinander folgenden A-Nukleotiden in dem Transkript kodiert.

12. Nukleinsäuremolekül nach Anspruch 11, wobei der zumindest eine Teil der Nukleinsäuresequenz (d) bei einer Transkription unter Kontrolle des Promotors (a) für eine Nukleotidsequenz von mindestens 80 aufeinander folgenden A-Nukleotiden in dem Transkript kodiert.

13. Nukleinsäuremolekül nach Anspruch 12, wobei der zumindest eine Teil der Nukleinsäuresequenz (d) bei einer Transkription unter Kontrolle des Promotors (a) für eine Nukleotidsequenz von mindestens 100 aufeinander folgenden A-Nukleotiden in dem Transkript kodiert.

14. Nukleinsäuremolekül nach Anspruch 13, wobei der zumindest eine Teil der Nukleinsäuresequenz (d) bei einer Transkription unter Kontrolle des Promotors (a) für eine Nukleotidsequenz von etwa 120 aufeinander folgenden A-Nukleotiden in dem Transkript kodiert.

15. Nukleinsäuremolekül nach einem der Ansprüche 9 bis 14, **dadurch gekennzeichnet, dass** es vor der Spaltung als kreisförmig geschlossenes Molekül und nach der Spaltung als lineares Molekül vorliegt.

16. Nukleinsäuremolekül nach einem der Ansprüche 9 bis 15, wobei die Spaltung mit Hilfe einer Restriktionsschnittstelle erfolgt.

17. Nukleinsäuremolekül nach Anspruch 16, wobei die Restriktionsschnittstelle eine Restriktionsschnittstelle für eine Restriktions-Endonuklease des Typs IIS ist.

18. Nukleinsäuremolekül nach Anspruch 17, wobei die Erkennungssequenz für die Restriktions-Endonuklease des Typs IIS stromabwärts und 5-26 Basenpaare entfernt vom 3'-Ende der Nukleinsäuresequenz (d) liegt.

19. Nukleinsäuremolekül nach Anspruch 18, wobei die Erkennungssequenz für die Restriktions-Endonuklease des Typs IIS stromabwärts und 24-26 Basenpaare entfernt vom 3'-Ende der Nukleinsäuresequenz (d) liegt.

20. Nukleinsäuremolekül nach einem der Ansprüche 1 bis 19, wobei das beta-Globin-Gen humanes beta-Globin-Gen ist.

21. Nukleinsäuremolekül nach einem der Ansprüche 1 bis 20, wobei die transkribierbare Nukleinsäuresequenz eine für ein Peptid oder Protein kodierende Nukleinsäuresequenz umfasst und die Nukleinsäuresequenz für ein Einbringen einer transkribierbaren Nukleinsäuresequenz eine multiple Klonierunesstelle ist.

22. Nukleinsäuremolekül nach einem der Ansprüche 1 bis 21, ferner umfassend ein oder mehrere Mitglieder ausgewählt aus der Gruppe bestehend aus: (i) einem Reportergen; (ii) einem selektierbaren Marker; und (iii) einem Replikationsursprung.

23. Nukleinsäuremolekül nach einem der Ansprüche 1 bis 22, wobei das Nukleinsäuremolekül in einer kreisförmig geschlossenen Konformation vorliegt.

24. Nukleinsäuremolekül nach einem der Ansprüche 1 bis 23, das insbesondere nach einer Linearisierung für die in vitro-Transkription von RNA, insbesondere mRNA, geeignet ist.

25. Nukleinsäuremolekül, das durch Linearisierung des Nukleinsäuremoleküls nach einem der Ansprüche 1 bis 24 erhältlich ist.

26. RNA, die durch Transkription, vorzugsweise in vitro-Transkription, mit einem Nukleinsäuremolekül nach einem der Ansprüche 1 bis 25 unter Kontrolle des Promotors (a) erhältlich ist, wobei in der RNA Transkripte der Nukleinsäuresequenzen (b), (c-1), (c-2) und gegebenenfalls (c-3) in einem gemeinsamen Transkript vorliegen.

27. Verfahren zur in vitro-Transkription eines ausgewählten RNA-Moleküls, um dessen Stabilität und/oder Translationseffizienz zu erhöhen, umfassend:
(i) Koppeln einer ersten Nukleinsäuresequenz (b-1) an dem 3'-Ende einer Nukleinsäuresequenz (a), die in das RNA-Molekül transkribierbar ist,
(ii) Koppeln einer zweiten Nukleinsäuresequenz (b-2) an dem 3'-Ende der ersten Nukleinsäuresequenz (b-1), und gegebenenfalls
(iii) Koppeln mindestens einer weiteren Nukleinsäuresequenz (b-3) an dem 3'-Ende der zweiten Nukleinsäuresequenz (b-2),
wobei die Nukleinsäuresequenzen (b-1), (b-2) und gegebenenfalls (b-3) ausgewählt sind aus der Gruppe bestehend aus:
(I) einer Nukleinsäuresequenz, die der 3'-nicht-translatierten Region eines Globin-Gens entspricht und
(II) einer Nukleinsäuresequenz, die zu der Nukleinsäuresequenz unter (I) mindestens 90% Identität aufweist,
wobei die Nukleinsäuresequenzen (b-1), (b-2) und gegebenenfalls (b-3) unabhängig voneinander von einem Gen abgeleitet sind, ausgewählt aus der Gruppe, bestehend aus alpha2-Globin-Gen, alphal-Globin-Gen, und beta-Globin-Gen, und
(iv) in vitro-Transkription der erhaltenen Nukleinsäure,
wobei die Nukleinsäuresequenzen (a), (b-1), (b-2) und gegebenenfalls (b-3) in ein gemeinsames Transkript transkribierbar sind und in dem gemeinsamen Transkript die von den Nukleinsäuresequenzen (b-1), (b-2) und gegebenenfalls (b-3) transkribierten Nukleinsäuresequenzen wirksam sind, die Translationseffizienz und/oder die Stabilität der von der transkribierbaren Nukleinsäuresequenz (a) transkribierten Nukleinsäuresequenz zu erhöhen.

28. Verfahren zur Translation eines ausgewählten mRNA-Moleküls, um dessen Expression zu erhöhen, umfassend:
(i) Koppeln einer ersten Nukleinsäuresequenz (b-1) an dem 3'-Ende einer Nukleinsäuresequenz (a), die in das mRNA-Molekül transkribierbar ist,
(ii) Koppeln einer zweiten Nukleinsäuresequenz (b-2) an dem 3'-Ende der ersten Nukleinsäuresequenz (b-1), und gegebenenfalls
(iii) Koppeln mindestens einer weiteren Nukleinsäuresequenz (b-3) an dem 3'-Ende der zweiten Nukleinsäuresequenz (b-2),
wobei die Nukleinsäuresequenzen (b-1), (b-2) und gegebenenfalls (b-3) ausgewählt sind aus der Gruppe bestehend aus:
(I) einer Nukleinsäuresequenz, die der 3'-nicht-translatierten Region eines Globin-Gens entspricht, oder
(II) einer Nukleinsäuresequenz, die zu der Nukleinsäuresequenz unter (I) mindestens 90% Identität aufweist,
wobei die Nukleinsäuresequenzen (b-1), (b-2) und gegebenenfalls (b-3) unabhängig voneinander von einem Gen abgeleitet sind, ausgewählt aus der Gruppe, bestehend aus alpha2-Globin-Gen, alphal-Globin-Gen, und beta-Globin-Gen, und
(iv) Translatieren der RNA, die durch Transkription der erhaltenen Nukleinsäure erhältlich ist,
wobei die Nukleinsäuresequenzen (a), (b-1), (b-2) und gegebenenfalls (b-3) in ein gemeinsames Transkript transkribierbar sind und in dem gemeinsamen Transkript die von den Nukleinsäuresequenzen (b-1), (b-2) und gegebenenfalls (b-3) transkribierten Nukleinsäuresequenzen wirksam sind, die Translationseffizienz und/oder die Stabilität der von der transkribierbaren Nukleinsäuresequenz (a) transkribierten Nukleinsäuresequenz zu erhöhen.

29. Verfahren nach Anspruch 28, wobei die Transkription in vitro erfolgt.

30. Verfahren nach einem der Ansprüche 27 bis 29, das ferner ein Koppeln einer Nukleinsäuresequenz (c), die bei einer Transkription für eine Nukleotidsequenz von mindestens 20 aufeinander folgenden A-Nukleotiden kodiert, an dem 3'-Ende der Nukleinsäuresequenz (b-2) oder gegebenenfalls der Nukleinsäuresequenz (b-3) umfasst.

31. Verfahren nach Anspruch 30, wobei die Nukleinsäuresequenzen (a), (b-1), (b-2), gegebenenfalls (b-3), und (c) in ein gemeinsames Transkript transkribierbar sind und in dem gemeinsamen Transkript die von den Nukleinsäuresequenzen (b-1), (b-2), gegebenenfalls (b-3) und (c) transkribierten Nukleinsäuresequenzen wirksam sind, die Translationseffizienz und/oder die Stabilität der von der Nukleinsäuresequenz (a) transkribierten Nukleinsäuresequenz zu erhöhen.

32. Verfahren nach Anspruch 30 oder 31, **dadurch gekennzeichnet, dass** es ferner vor einer Transkription der erhaltenen Nukleinsäure eine Spaltung innerhalb der Nukleinsäuresequenz (c) umfasst, so dass bei einer Transkription der so erhaltenen Nukleinsäure ein Transkript erzeugt wird, das die von den Nukleinsäuresequenzen (a), (b-1), (b-2) und gegebenenfalls (b-3) transkribierten Nukleinsäuresequenzen und an seinem 3'-Ende eine Nukleotidsequenz von mindestens 20 aufeinander folgenden A-Nukleotiden aufweist, wobei das Transkript als 3'-terminales Nukleotid ein A-Nukleotid aus der Nukleotidsequenz von mindestens 20 aufeinander folgenden A-Nukleotiden aufweist.

33. Verfahren nach einem der Ansprüche 27 bis 32, wobei das beta-Globin-Gen humanes beta-Globin-Gen ist.

34. Verfahren nach Anspruch 32, wobei die Spaltung mit Hilfe einer Restriktionsschnittstelle erfolgt.

35. Verfahren nach Anspruch 34, wobei die Restriktionsschnittstelle eine Restriktionsschnittstelle für eine Restriktions-Endonuklease des Typs IIS ist.

36. Verfahren nach Anspruch 35, wobei die Erkennungssequenz für die Restriktions-Endonuklease des Typs IIS stromabwärts und 5-26 Basenpaare entfernt vom 3'-Ende der Nukleinsäuresequenz, die bei einer Transkription für eine Nukleotidsequenz von mindestens 20 aufeinander folgenden A-Nukleotiden kodiert, liegt.

37. Verfahren nach Anspruch 36, wobei die Erkennungssequenz für die Restriktions-Endonuklease des Typs IIS stromabwärts und 24-26 Basenpaare entfernt vom 3'-Ende der Nukleinsäuresequenz, die bei einer Transkription für eine Nukleotidsequenz von mindestens 20 aufeinander folgenden A-Nukleotiden kodiert, liegt.

38. RNA, die durch ein Verfahren zur in vitro-Transkription eines ausgewählten RNA-Moleküls nach einem der Ansprüche 27 und 30-37 erhältlich ist, wobei in der RNA Transkripte der Nukleinsäuresequenzen (a), (b- 1), (b-2) und gegebenenfalls (b-3) in einem gemeinsamen Transkript vorliegen.

39. Verwendung der RNA nach Anspruch 26 oder 38 zur Transfektion einer Wirtszelle.

40. Verwendung nach Anspruch 39, wobei die Wirtszelle eine Antigen-präsentierende Zelle, insbesondere eine dendritische Zelle, ein Monozyt oder ein Makrophage, ist.

## Claims

1. A nucleic acid molecule comprising in the 5' → 3' direction of transcription:
(a) a promoter,
(b) a transcribable nucleic acid sequence or a nucleic acid sequence for introducing a transcribable nucleic acid sequence,
(c-1) a first nucleic acid sequence,
(c-2) a second nucleic acid sequence and, where appropriate,
(c-3) at least one further nucleic acid sequence, wherein the nucleic acid sequences (c-1), (c-2) and, where appropriate, (c-3) are selected from the group consisting of:
(I) a nucleic acid sequence which corresponds to the 3'-untranslated region of a globin gene and
(II) a nucleic acid sequence which is at least 90% identical to the nucleic acid sequence under (I), wherein the nucleic acid sequences (c-1), (c-2) and, where appropriate, (c-3) are independently of one another derived from a gene selected from the group consisting of alpha2-globin gene, alphal-globin gene and beta-globin gene and
wherein the nucleic acid sequences (b), (c-1), (c-2) and, where appropriate, (c-3) under the control of the promoter (a) can be transcribed to give a common transcript in which the nucleic acid sequences transcribed from the nucleic acid sequences (c-1), (c-2) and, where appropriate, (c-3) are active so as to increase the translation efficiency and/or the stability of the nucleic acid sequence transcribed from the transcribable nucleic acid sequence (b).

2. The nucleic acid molecule as claimed in claim 1, wherein the nucleic acid sequences (c-1), (c-2) and, where appropriate, (c-3) can be identical or different.

3. The nucleic acid molecule as claimed in claim 1 or 2, which further comprises (d) a nucleic acid sequence which, when transcribed under the control of the promoter (a), codes for a nucleotide sequence of at least 20 consecutive A nucleotides in the transcript.

4. The nucleic acid molecule as claimed in claim 3, wherein the nucleic acid sequences (b), (c-1), (c-2), where appropriate (c-3), and (d) under the control of the promoter (a) can be transcribed to give a common transcript in which the nucleic acid sequences transcribed from the nucleic acid sequences (c-1), (c-2), where appropriate, (c-3) and (d) are active so as to increase the translation efficiency and/or the stability of the nucleic acid sequence transcribed from the transcribable nucleic acid sequence (b).

5. The nucleic acid molecule as claimed in claim 3 or 4, wherein the nucleic acid sequence (d), when transcribed under the control of the promoter (a), codes for a nucleotide sequence of at least 40 consecutive A nucleotides in the transcript.

6. The nucleic acid molecule as claimed in claim 5, wherein the nucleic acid sequence (d), when transcribed under the control of the promoter (a), codes for a nucleotide sequence of at least 80 consecutive A nucleotides in the transcript.

7. The nucleic acid molecule as claimed in claim 6, wherein the nucleic acid sequence (d), when transcribed under the control of the promoter (a), codes for a nucleotide sequence of at least 100 consecutive A nucleotides in the transcript.

8. The nucleic acid molecule as claimed in claim 7, wherein the nucleic acid sequence (d), when transcribed under the control of the promoter (a), codes for a nucleotide sequence of about 120 consecutive A nucleotides in the transcript.

9. The nucleic acid molecule as claimed in any of claims 3 to 8, **characterized in that** it can be cleaved, preferably enzymatically or in another biochemical way, within the nucleic acid sequence (d) in such a way that said cleavage results in a nucleic acid molecule which comprises, in the 5' → 3' direction of transcription, the promoter (a), the nucleic acid sequence (b), the nucleic acid sequences (c-1), (c-2), where appropriate, (c-3) and at least a part of the nucleic acid sequence (d), wherein the at least a part of the nucleic acid sequence (d), when transcribed under the control of the promoter (a), codes for a nucleotide sequence of at least 20 consecutive A nucleotides in the transcript and wherein in the transcript the 3'-terminal nucleotide is an A nucleotide of said nucleotide sequence of at least 20 consecutive A nucleotides.

10. The nucleic acid molecule as claimed in claim 9, wherein, after cleavage, said nucleic acid molecule, at the end of the strand that serves as template for the nucleotide sequence of at least 20 consecutive A nucleotides, has a T nucleotide which is part of the nucleotide sequence which serves as template for said nucleotide sequence of at least 20 consecutive A nucleotides in the transcript.

11. The nucleic acid molecule as claimed in claim 9 or 10, wherein the at least a part of the nucleic acid sequence (d), when transcribed under the control of the promoter (a), codes for a nucleotide sequence of at least 40 consecutive A nucleotides in the transcript.

12. The nucleic acid molecule as claimed in claim 11, wherein the at least a part of the nucleic acid sequence (d), when transcribed under the control of the promoter (a), codes for a nucleotide sequence of at least 80 consecutive A nucleotides in the transcript.

13. The nucleic acid molecule as claimed in claim 12, wherein the at least a part of the nucleic acid sequence (d), when transcribed under the control of the promoter (a), codes for a nucleotide sequence of at least 100 consecutive A nucleotides in the transcript.

14. The nucleic acid molecule as claimed in claim 13, wherein the at least a part of the nucleic acid sequence (d), when transcribed under the control of the promoter (a), codes for a nucleotide sequence of about 120 consecutive A nucleotides in the transcript.

15. The nucleic acid molecule as claimed in any of claims 9 to 14, **characterized in that** it is a closed circular molecule prior to cleavage and a linear molecule after cleavage.

16. The nucleic acid molecule as claimed in any of claims 9 to 15, wherein cleavage is carried out with the aid of a restriction cleavage site.

17. The nucleic acid molecule as claimed in claim 16, wherein the restriction cleavage site is a restriction cleavage site for a type IIS restriction endonuclease.

18. The nucleic acid molecule as claimed in claim 17, wherein the recognition sequence for the type IIS restriction endonuclease is located 5-26 base pairs downstream of the 3' end of the nucleic acid sequence (d).

19. The nucleic acid molecule as claimed in claim 18, wherein the recognition sequence for the type IIS restriction endonuclease is located 24-26 base pairs downstream of the 3' end of the nucleic acid sequence (d).

20. The nucleic acid molecule as claimed in any of claims 1 to 19, wherein the beta-globin gene is human beta-globin gene.

21. The nucleic acid molecule as claimed in any of claims 1 to 20, wherein the transcribable nucleic acid sequence comprises a nucleic acid sequence coding for a peptide or protein and the nucleic acid sequence for introducing a transcribable nucleic acid sequence is a multiple cloning site.

22. The nucleic acid molecule as claimed in any of claims 1 to 21, further comprising one or more members selected from the group consisting of: (i) a reporter gene; (ii) a selectable marker; and (iii) an origin of replication.

23. The nucleic acid molecule as claimed in any of claims 1 to 22, wherein the nucleic acid molecule is in a closed circular conformation.

24. The nucleic acid molecule as claimed in any of claims 1 to 23, which is suitable, in particular after linearization, for in vitro transcription of RNA, in particular mRNA.

25. A nucleic acid molecule which is obtainable by linearization of the nucleic acid molecule as claimed in any of claims 1 to 24.

26. An RNA which is obtainable by transcription, preferably in vitro transcription, with a nucleic acid molecule as claimed in any of claims 1 to 25 under the control of the promoter (a), wherein in the RNA transcripts of the nucleic acid sequences (b), (c-1), (c-2) and, where appropriate, (c-3) are present in a common transcript.

27. A method of transcribing in vitro a selected RNA molecule in order to increase its stability and/or translation efficiency, comprising:
(i) coupling a first nucleic acid sequence (b-1) at the 3' end of a nucleic acid sequence (a) which can be transcribed to give said RNA molecule,
(ii) coupling a second nucleic acid sequence (b-2) at the 3' end of said first nucleic acid sequence (b-1), and, where appropriate,
(iii) coupling at least one further nucleic acid sequence (b-3) at the 3' end of said second nucleic acid sequence (b-2),
wherein the nucleic acid sequences (b-1), (b-2) and, where appropriate, (b-3) are selected from the group consisting of:
(I) a nucleic acid sequence which corresponds to the 3'-untranslated region of a globin gene and
(II) a nucleic acid sequence which is at least 90% identical to the nucleic acid sequence under (I), wherein the nucleic acid sequences (b-1), (b-2) and, where appropriate, (b-3) are independently of one another derived from a gene selected from the group consisting of alpha2-globin gene, alphal-globin gene and beta-globin gene and
(iv) transcribing in vitro the nucleic acid obtained wherein the nucleic acid sequences (a), (b-1), (b-2) and, where appropriate, (b-3) can be transcribed to give a common transcript in which the nucleic acid sequences transcribed from the nucleic acid sequences (b-1), (b-2) and, where appropriate, (b-3) are active so as to increase the translation efficiency and/or the stability of the nucleic acid sequence transcribed from the transcribable nucleic acid sequence (a).

28. A method of translating a selected mRNA molecule in order to increase expression thereof, comprising:
(i) coupling a first nucleic acid sequence (b-1) at the 3' end of a nucleic acid sequence (a) which can be transcribed to give said mRNA molecule,
(ii) coupling a second nucleic acid sequence (b-2) at the 3' end of said first nucleic acid sequence (b-1), and, where appropriate,
(iii) coupling at least one further nucleic acid sequence (b-3) at the 3' end of said second nucleic acid sequence (b-2),
wherein the nucleic acid sequences (b-1), (b-2) and, where appropriate, (b-3) are selected from the group consisting of:
(I) a nucleic acid sequence which corresponds to the 3'-untranslated region of a globin gene and
(II) a nucleic acid sequence which is at least 90% identical to the nucleic acid sequence under (I), wherein the nucleic acid sequences (b-1), (b-2) and, where appropriate, (b-3) are independently of one another derived from a gene selected from the group consisting of alpha2-globin gene, alpha1-globin gene and beta-globin gene and
(iv) translating the mRNA which is obtainable by transcribing the nucleic acid obtained,
wherein the nucleic acid sequences (a), (b-1), (b-2) and, where appropriate, (b-3) can be transcribed to give a common transcript in which the nucleic acid sequences transcribed from the nucleic acid sequences (b-1), (b-2) and, where appropriate, (b-3) are active so as to increase the translation efficiency and/or the stability of the nucleic acid sequence transcribed from the transcribable nucleic acid sequence (a)

29. The method as claimed in claim 28, wherein transcription is carried out in vitro.

30. The method as claimed in any of claims 27 to 29, which further comprises coupling a nucleic acid sequence (c) which, when transcribed, codes for a nucleotide sequence of at least 20 consecutive A nucleotides, at the 3' end of the nucleic acid sequence (b-2) or, where appropriate, of the nucleic acid sequence (b-3).

31. The method as claimed in claim 30, wherein the nucleic acid sequences (a), (b-1), (b-2) and, where appropriate, (b-3), and (c) can be transcribed to give a common transcript in which the nucleic acid sequences transcribed from the nucleic acid sequences (b-1), (b-2), where appropriate, (b-3) and (c) are active so as to increase the translation efficiency and/or the stability of the nucleic acid sequence transcribed from the nucleic acid sequence (a).

32. The method as claimed in claim 30 or 31, **characterized in that** it further comprises, prior to transcription of the nucleic acid obtained, cleavage within the nucleic acid sequence (c) in such a way that transcription of the nucleic acid obtained in this way generates a transcript which has the nucleic acid sequences transcribed from the nucleic acid sequences (a), (b-1), (b-2) and, where appropriate, (b-3) and a 3'-terminal nucleotide sequence of at least 20 consecutive A nucleotides, wherein the 3'-terminal nucleotide of said transcript is an A nucleotide of the nucleotide sequence of at least 20 consecutive A nucleotides.

33. The method as claimed in any of claims 27 to 32, wherein the beta-globin gene is human beta-globin gene.

34. The method as claimed in claim 32, wherein cleavage is carried out with the aid of a restriction cleavage site.

35. The method as claimed in claim 34, wherein the restriction cleavage site is a restriction cleavage site for a type IIS restriction endonuclease.

36. The method as claimed in claim 35, wherein the recognition sequence for the type IIS restriction endonuclease is located 5-26 base pairs downstream of the 3' end of the nucleic acid sequence which, when transcribed, codes for a nucleotide sequence of at least 20 consecutive A nucleotides.

37. The method as claimed in claim 36, wherein the recognition sequence for the type IIS restriction endonuclease is located 24-26 base pairs downstream of the 3' end of the nucleic acid sequence which, when transcribed, codes for a nucleotide sequence of at least 20 consecutive A nucleotides.

38. An RNA obtainable by a method of transcribing in vitro a selected RNA molecule as claimed in any of claims 27 and 30-37, wherein in the RNA transcripts of the nucleic acid sequences (a), (b-1), (b-2) and, where appropriate, (b-3) are present in a common transcript.

39. The use of the RNA as claimed in claim 26 or 38 for transfecting a host cell.

40. The use as claimed in claim 39, wherein the host cell is an antigen-presenting cell, in particular a dendritic cell, a monocyte or a macrophage.

## Revendications

1. Molécule d'acide nucléique, comprenant dans la direction de transcription 5'Π3':
(a) un promoteur,
(b) une séquence d'acide nucléique transcriptible ou une séquence d'acide nucléique pour une introduction d'une séquence d'acide nucléique transcriptible,
(c-1) une première séquence d'acide nucléique,
(c-2) une deuxième séquence d'acide nucléique, et en option
(c-3) au moins une autre séquence d'acide nucléique,
tandis que les séquences d'acide nucléique (c-1), (c-2) et en option (c-3) sont choisies dans le groupe consistant en:
(I) une séquence d'acide nucléique qui correspond à la région 3'-non-traduite d'un gène de globine, et
(II) une séquence d'acide nucléique qui présente au moins 90% d'identité par rapport à la séquence d'acide nucléique sous (I),
tandis que les séquences d'acide nucléique (c-1), (c-2) et en option (c-3) sont dérivées indépendamment l'une de l'autre d'un gène choisi dans le groupe consistant en gène d'alpha2-globine, gène d'alpha1-globine, et gène de bêta-globine, et
tandis que les séquences d'acide nucléique (b), (c-1), (c-2) et en option (c-3) sont aptes à être transcrites sous le contrôle du promoteur (a) dans un transcrit commun, et que dans le transcrit commun les séquences d'acide nucléique transcrites à partir des séquences d'acide nucléique (c-1), c-2) et en option (c-3) sont actives pour augmenter l'efficacité de traduction et/ou la stabilité de la séquence d'acide nucléique transcrite à partir de la séquence d'acide nucléique transcriptible (b).

2. Molécule d'acide nucléique selon la revendication 1, dans laquelle les séquences d'acide nucléique (c-1), (c-2) et en option (c-3) peuvent être identiques ou différentes.

3. Molécule d'acide nucléique selon la revendication 1 ou 2, qui comprend en outre une séquence d'acide nucléique qui code lors d'une transcription sous le contrôle du promoteur (a) pour une séquence nucléotidique d'au moins 20 nucléotides A consécutifs dans le transcrit.

4. Molécule d'acide nucléique selon la revendication 3, dans laquelle les séquences d'acide nucléique (b), (c-1), (c-2), en option (c-3), et (d) sont transcriptibles sous le contrôle du promoteur (a) en un transcrit commun et dans le transcrit commun les séquences d'acide nucléique transcrites à partir des séquences d'acide nucléique (c-1), (c-2), en option (c-3) et (d) sont actives pour augmenter l'efficacité de traduction et/ou la stabilité de la séquence d'acide nucléique transcrite à partir de la séquence d'acide nucléique transcriptible (b).

5. Molécule d'acide nucléique selon la revendication 3 ou 4, dans laquelle la séquence d'acide nucléique (d) code, lors d'une transcription sous le contrôle du promoteur (a), pour une séquence nucléotidique d'au moins 40 nucléotides A consécutifs dans le transcrit.

6. Molécule d'acide nucléique selon la revendication 5, dans laquelle la séquence d'acide nucléique (d) code, lors d'une transcription sous le contrôle du promoteur (a), pour une séquence nucléotidique d'au moins 80 nucléotides A consécutifs dans le transcrit.

7. Molécule d'acide nucléique selon la revendication 6, dans laquelle la séquence d'acide nucléique (d) code, lors d'une transcription sous le contrôle du promoteur (a), pour une séquence nucléotidique d'au moins 100 nucléotides A consécutifs dans le transcrit.

8. Molécule d'acide nucléique selon la revendication 7, dans laquelle la séquence d'acide nucléique (d) code, lors d'une transcription sous le contrôle du promoteur (a), pour une séquence nucléotidique d'environ 120 nucléotides A consécutifs dans le transcrit.

9. Molécule d'acide nucléique selon l'une des revendications 3 à 8, **caractérisée en ce qu'**elle peut être scindée à l'intérieur de la séquence d'acide nucléique (d), de préférence par voie enzymatique ou encore biochimique, de telle manière que sous l'effet de la coupure il se forme une molécule d'acide nucléique qui comprend, dans la direction de transcription 5'Π 3', le promoteur (a), la séquence d'acide nucléique (b), les séquences d'acide nucléique (c-1), (c-2), en option (c-3), et au moins une partie de la séquence d'acide nucléique (d), tandis que la au moins une partie de la séquence d'acide nucléique (d) code, lors d'une transcription sous le contrôle du promoteur (a), pour une séquence nucléotidique d'au moins 20 nucléotides A consécutifs dans le transcrit, et que le transcrit présente comme nucléotide 3'-terminal un nucléotide A provenant de la séquence nucléotidique d'au moins 20 nucléotides A consécutifs.

10. Molécule d'acide nucléique selon la revendication 9, dans laquelle la molécule d'acide nucléique, après la coupure à l'extrémité du brin qui sert de matrice pour la séquence nucléotidique d'au moins 20 nucléotides A consécutifs, présente un nucléotide T qui fait partie de la séquence nucléotidique qui sert de matrice pour la séquence nucléotidique d'au moins 20 nucléotides A consécutifs dans le transcrit.

11. Molécule d'acide nucléique selon la revendication 9 ou 10, dans laquelle la au moins une partie de la séquence d'acide nucléique (d) code, lors d'une transcription sous le contrôle du promoteur (a), pour une séquence nucléotidique d'au moins 40 nucléotides A consécutifs dans le transcrit.

12. Molécule d'acide nucléique selon la revendication 11, dans laquelle la au moins une partie de la séquence d'acide nucléique (d) code, lors d'une transcription sous le contrôle du promoteur (a), pour une séquence nucléotidique d'au moins 80 nucléotides A consécutifs dans le transcrit.

13. Molécule d'acide nucléique selon la revendication 12, dans laquelle la au moins une partie de la séquence d'acide nucléique (d) code, lors d'une transcription sous le contrôle du promoteur (a), pour une séquence nucléotidique d'au moins 100 nucléotides A consécutifs dans le transcrit.

14. Molécule d'acide nucléique selon la revendication 13, dans laquelle la au moins une partie de la séquence d'acide nucléique (d) code, lors d'une transcription sous le contrôle du promoteur (a), pour une séquence nucléotidique d'environ 120 nucléotides A consécutifs dans le transcrit.

15. Molécule d'acide nucléique selon l'une des revendications 9 à 14, **caractérisée en ce qu'**elle se présente avant la coupure à l'état de molécule en boucle fermée et après la coupure sous forme de molécule linéaire.

16. Molécule d'acide nucléique selon l'une des revendications 9 à 15, dans laquelle la coupure s'effectue au moyen d'un site de coupure par restriction.

17. Molécule selon la revendication 16, dans laquelle le site de coupure par restriction est un site de coupure par restriction pour une endonucléase de restriction de type IIS.

18. Molécule d'acide nucléique selon la revendication 17, dans laquelle la séquence de reconnaissance pour l'endonucléase de restriction de type IIS se situe en aval et à distance de 5-26 paires de bases de l'extrémité 3' de la séquence d'acide nucléique (d).

19. Molécule d'acide nucléique selon la revendication 18, dans laquelle la séquence de reconnaissance pour l'endonucléase de restriction de type IIS se situe en aval et à distance de 24-26 paires de bases de l'extrémité 3' de la séquence d'acide nucléique (d).

20. Molécule d'acide nucléique selon l'une des revendications 1 à 19, dans laquelle le gène de bêta-globine est un gène de bêta-globine humain.

21. Molécule d'acide nucléique selon l'une des revendications 1 à 20, dans laquelle la séquence d'acide nucléique transcriptible comprend une séquence d'acide nucléique codant pour un peptide ou une protéine et la séquence d'acide nucléique pour l'introduction d'une séquence d'acide nucléique transcriptible est un site de clonage multiple.

22. Molécule d'acide nucléique selon l'une des revendications 1 à 21, comprenant en outre un ou plusieurs éléments choisis dans le groupe consistant en: (i) un gène reporter; (ii) un marqueur sélectionnable; et (iii) une origine de réplication.

23. Molécule d'acide nucléique selon l'une des revendications 1 à 22, dans laquelle la molécule d'acide nucléique se présente dans une conformation en boucle fermée.

24. Molécule d'acide nucléique selon l'une des revendications 1 à 23, qui convient, en particulier après une linéarisation, pour la transcription in vitro d'ARN, en particulier d'ARNm.

25. Molécule d'acide nucléique, qui peut être obtenue par linéarisation de la molécule d'acide nucléique selon l'une des revendications 1 à 24.

26. ARN qui peut être obtenu par transcription, de préférence par transcription in vitro, avec une molécule d'acide nucléique selon l'une des revendications 1 à 25 sous le contrôle du promoteur (a), tandis que dans l'ARN des transcrits des séquences d'acide nucléique (b), (c-1), (c-2) et en option (c-3) sont présents dans un transcrit commun.

27. Procédé pour la transcription in vitro d'une molécule d'ARN sélectionnée, pour augmenter la stabilité et/ou l'efficacité de traduction de celle-ci, comprenant:
(i) le couplage d'une première séquence d'acide nucléique (b-1) à l'extrémité 3' d'une séquence d'acide nucléique (a), qui est transcriptible dans la molécule d'ARN,
(ii) le couplage d'une deuxième séquence d'acide nucléique (b-2) à l'extrémité 3' de la première séquence d'acide nucléique (b-1), et en option
(iii) le couplage d'au moins une autre séquence d'acide nucléique (b-3) à l'extrémité 3' de la deuxième séquence d'acide nucléique (b-2),
tandis que les séquences d'acide nucléique (b-1), (b-2) et en option (b-3) sont choisies dans le groupe consistant en:
(I) une séquence d'acide nucléique qui correspond à la région 3'-non-traduite d'un gène de globine, et
(II) une séquence d'acide nucléique qui présente au moins 90% d'identité vis-à-vis de la séquence d'acide nucléique sous (I),
tandis que les séquences d'acide nucléique (b-1), (b-2) et en option (b-3) sont dérivées indépendamment l'une de l'autre d'un gène choisi dans le groupe consistant en gène d'alpha2-globine, gène d'alpha1-globine, et gène de bêta-globine, et
(iv) la transcription in vitro de l'acide nucléique obtenu,
tandis que les séquences d'acide nucléique (a), (b-1), (b-2) et en option (b-3) sont aptes à être transcrites en un transcrit commun, et que dans le transcrit commun les séquences d'acide nucléique transcrites à partir des séquences d'acide nucléique (b-1), b-2) et en option (b-3) sont actives pour augmenter l'efficacité de traduction et/ou la stabilité de la séquence d'acide nucléique transcrite à partir de la séquence d'acide nucléique transcriptible (a).

28. Procédé pour la traduction d'une molécule d'ARNm sélectionnée, pour augmenter l'expression de celle-ci, comprenant:
(i) le couplage d'une première séquence d'acide nucléique (b-1) à l'extrémité 3' d'une séquence d'acide nucléique (a), qui est transcriptible dans la molécule d'ARNm,
(ii) le couplage d'une deuxième séquence d'acide nucléique (b-2) à l'extrémité 3' de la première séquence d'acide nucléique (b-1), et en option
(iii) le couplage d'au moins une autre séquence d'acide nucléique (b-3) à l'extrémité 3' de la deuxième séquence d'acide nucléique (b-2),
tandis que les séquences d'acide nucléique (b-1), (b-2) et en option (b-3) sont choisies dans le groupe consistant en:
(I) une séquence d'acide nucléique qui correspond à la région 3'-non-traduite d'un gène de globine, ou
(II) une séquence d'acide nucléique qui présente au moins 90% d'identité vis-à-vis de la séquence d'acide nucléique sous (I),
tandis que les séquences d'acide nucléique (b-1), (b-2) et en option (b-3) sont dérivées indépendamment l'une de l'autre d'un gène choisi dans le groupe consistant en gène d'alpha2-globine, gène d'alpha1-globine, et gène de bêta-globine, et
(iv) la traduction de l'ARNm, qui peut être obtenu par transcription de l'acide nucléique obtenu,
tandis que les séquences d'acide nucléique (a), (b-1), (b-2) et en option (b-3) sont aptes à être transcrites en un transcrit commun, et que dans le transcrit commun les séquences d'acide nucléique transcrites à partir des séquences d'acide nucléique (b-1), b-2) et en option (b-3) sont actives pour augmenter l'efficacité de traduction et/ou la stabilité de la séquence d'acide nucléique transcrite à partir de la séquence d'acide nucléique transcriptible (a).

29. Procédé selon la revendication 28, dans lequel la transcription est effectuée in vitro.

30. Procédé selon l'une des revendications 27 à 29, qui comprend en outre un couplage d'une séquence d'acide nucléique (c), qui code lors d'une transcription pour une séquence nucléotidique d'au moins 20 nucléotides A consécutifs, à l'extrémité 3' de la séquence d'acide nucléique (b-2) ou en option de la séquence d'acide nucléique (b-3).

31. Procédé selon la revendication 30, dans lequel les séquences d'acide nucléique (a), (b-1), (b-2), en option (b-3), et (c) sont aptes à être transcrites dans un transcrit commun, et dans le transcrit commun les séquences d'acide nucléique transcrites à partir des séquences d'acide nucléique (b-1), (b-2) en option (b-3) et (c) sont actives pour augmenter l'efficacité de traduction et/ou la stabilité de la séquence d'acide nucléique transcrite à partir de la séquence d'acide nucléique (a).

32. Procédé selon la revendication 30 ou 31, **caractérisé en ce qu'**il comprend en outre, avant une transcription de l'acide nucléique obtenu, une coupure à l'intérieur de la séquence d'acide nucléique (c), de telle manière que lors d'une transcription de l'acide nucléique ainsi obtenu il se forme un transcrit qui présente les séquences d'acide nucléique transcrites à partir des séquences d'acide nucléique (a), (b-1), (b-2) et en option (b-3) et présente à son extrémité 3' une séquence nucléotidique d'au moins 20 nucléotides A consécutifs, tandis que le transcrit présente comme nucléotide 3'-terminal un nucléotide A provenant de la séquence nucléotidique d'au moins 20 nucléotides A consécutifs.

33. Procédé selon l'une des revendications 27 à 32, dans lequel le gène de bêta-globine et un gène de bêta-globine humain.

34. Procédé selon la revendication 32, dans lequel la coupure est effectuée au moyen d'un site de coupure par restriction.

35. Procédé selon la revendication 34, dans lequel le site de coupure par restriction est un site de coupure par restriction pour une endonucléase de restriction du type IIS.

36. Procédé selon la revendication 35, dans lequel la séquence de reconnaissance pour l'endonucléase de restriction du type IIS se situe en aval et éloignée de 5-26 paires de bases de l'extrémité 3' de la séquence d'acide nucléique qui, lors d'une transcription, code pour une séquence nucléotidique d'au moins 20 nucléotides A consécutifs.

37. Procédé selon la revendication 36, dans lequel la séquence de reconnaissance pour l'endonucléase de restriction du type IIS se situe en aval et éloignée de 24-26 paires de bases de l'extrémité 3' de la séquence d'acide nucléique qui, lors d'une transcription, code pour une séquence nucléotidique d'au moins 20 nucléotides A consécutifs.

38. ARN, qui peut être obtenu par un procédé pour la transcription in vitro d'une molécule d'ARN sélectionnée selon l'une des revendications 27 et 30-37, tandis que dans l'ARN les transcrits des séquences d'acide nucléique (a), (b-1), (b-2) et en option (b-3) sont présentes dans un transcrit commun.

39. Utilisation de l'ARN selon la revendication 26 ou 38 pour la transfection d'une cellule hôte.

40. Utilisation selon la revendication 39, dans laquelle la cellule hôte est une cellule présentant l'antigène, en particulier une cellule dendritique, un monocyte ou un macrophage.
